# EUROPEAN PATENT APPLICATION

(11) **EP 4 428 137 A1**
(43) Date of publication of application: **11.09.2024**
(21) Application number: 22886159.7
(22) Date of filing: 31.10.2022
(51) Int. Cl.: C07D 495/04, A61K 31/519, A61P 35/00, A61P 15/00, A61P 15/08, A61P 15/18, A61P 13/08

(54) **GONADOTROPIN-RELEASING HORMONE ANTAGONIST, AND PREPARATION METHOD THEREFOR AND USE THEREOF**

(30) Priority: 01.11.2021 CN 202111281363; 19.11.2021 CN 202111375858
(71) Applicant: Shandong Luye Pharmaceutical Co., Ltd., Shandong 264670 (CN)
(72) Inventor: TIAN, Jingwei, Shandong 264670 (CN); ZOU, Fangxia, Shandong 264670 (CN); YU, Dawei, Shandong 264670 (CN); YE, Liang, Shandong 264670 (CN); LU, Jing, Shandong 264670 (CN); ZHANG, Rui, Shandong 264670 (CN); ZHANG, Jianzhao, Shandong 264670 (CN); WANG, Wenyan, Shandong 264670 (CN)
(74) Representative: karo IP
(86) International application number: PCT/CN2022/128588
(87) International publication number: WO 2023/072284

(57) **Abstract**

The present invention relates to a gonadotropin-releasing hormone antagonist, which has an antagonistic activity against gonadotropin-releasing hormone and can be used for the prevention or treatment of a sex hormone-dependent disease, such as benign prostatic hypertrophy, hysteromyoma, endometriosis, metrofibroma, precocious puberty, amenorrhea, premenstrual syndrome, or dysmenorrhea, or a prodrug thereof, or a pharmaceutically acceptable salt thereof, or a hydrate or solvate thereof, and a pharmaceutical composition comprising the same.

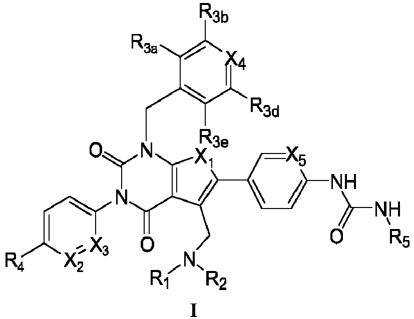

## Description

### TECHNICAL FIELD

The present invention belongs to the technical field of pharmaceuticals, and relates to a compound and a pharmaceutical composition for preventing or treating a sex hormone-dependent disease, and a method of use and use thereof. In particular, the present invention relates to a substituted pyrimidinedione that can be used as a gonadotropin releasing hormone receptor antagonist and use thereof.

### BACKGROUND

Secretion of anterior pituitary hormones is feedback-controlled by peripheral hormones secreted from target organs of the respective hormones and by secretion-regulating hormones from hypothalamus, which is the upper central organ of anterior pituitary (these hormones are collectively called "hypothalamic hormones"). Currently, it has been confirmed that there are nine hormones including, for example, thyrotropin-releasing hormone (TRH) and gonadotropin-releasing hormone (GnRH) as hypothalamic hormones. These hypothalamic hormones are thought to exhibit their effects via receptors that are thought to be present in the anterior pituitary. Efforts have been made to find receptor-gene expression specific to these hormones. Thus, antagonists or agonists that act specifically and selectively on these receptors should control the action of hypothalamic hormones and the secretion of anterior pituitary hormones. Therefore, such antagonists or agonists are expected to be useful for the prevention or treatment of anterior pituitary hormone-dependent diseases.

As agents for inhibiting GnRH receptors and the function of GnRH receptors, superagonists have been used as agents for treating sex hormone-dependent diseases such as prostate cancer, breast cancer, and endometriosis. GnRH receptor superagonists bind to the GnRH receptor and exert an initial transient gonadotropin secretion-stimulating effect, a so-called "flare-up" phenomenon, and then inhibit the function by inducing a depletion of the gonadotropin and the down-regulation of the GnRH receptor. Therefore, since GnRH receptor superagonists initially promote the secretion of gonadotropins, there is a defect that the disease is transiently exacerbated. In contrast, the mechanism of inhibition of GnRH receptor antagonists (hereinafter referred to as "GnRH antagonists") is the inhibition of binding to the GnRH receptor, and therefore, it is expected that the inhibitory effect will be exerted rapidly without secreting gonadotropins.

The compounds having GnRH antagonistic activity known so far are mostly peptide compounds such as GnRH-derived linear peptides (US5,140,009 and US5,171,835), bicyclic peptide derivatives (Journal of Medicinal Chemistry, Vol.36, pp. 3265-3273 (1993)), decapeptide compounds modified at position 5 or 6 (WO9846634A1), and decapeptide compounds modified at position 8 (EP0277829B1). There are many problems to be solved for peptide compounds, which are associated with oral absorbability, dosage form, dose volume, drug stability, sustaining action, metabolic stability, and the like. In view of the limitations of peptidic GnRH receptor antagonists, some non-peptidic GnRH receptor antagonists have been proposed and introduced into the development, clinical trial, or marketing stage. For example, Elagolix (also known as NBI-56418 or ABT-620) is a small molecule GnRH receptor antagonist developed by Abbott and Neurocrine Biosciences Inc, which is currently in the clinical Phase III stage and is mainly applied for the treatment of endometriosis (stage III) and uterine fibroids (stage II).

Relugolix, also known as TAK-385, is an oral antagonist of the small molecule GnRH receptor developed by Takada Pharmaceutical in Japan, for the treatment of endometriosis, uterine fibroids, and prostate cancer.

Although a large amount of significant research has been conducted in this field, there is a need for further research to develop more effective small molecule GnRH receptor antagonists.

### SUMMARY

The present invention provides a compound of formula (I), and a pharmaceutically acceptable salt or a stereoisomer thereof, 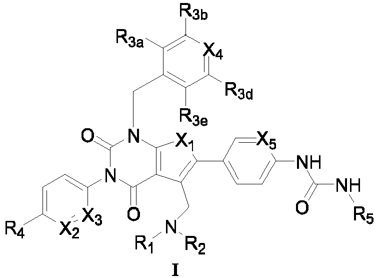 wherein:
X₁ is independently selected from O or S;
X₂ and X₃ are each independently selected from CR₄ or N;
X₄ is independently selected from CR_{3c} or N;
X₅ is independently selected from CR₆ or N;
R₁ is independently selected from H, D, C₁-C₆ alkyl, C₃-C₆ cycloalkyl, and C₁-C₆ haloalkyl;
R₂ is independently selected from H, D, C₁-C₆ alkyl, and C₁-C₆ alkyl substituted with R₂ₐ-O-;
R₂ₐ is independently selected from C₁-C₆ haloalkyl;
R₃ₐ, R_{3b}, R_{3c}, R_{3d}, and R₃ₑ are each independently selected from H, D, halogen, C₁-C₆ alkyl, C₁-C₆ haloalkyl, and C₃-C₆ cycloalkyl;
R₄ is independently selected from H, D, halogen, -CN, -NO₂, -NH₂, -OH, -SH, -COOH, C₁-C₆ alkyl, C₁-C₆ haloalkyl, C₁-C₆ alkoxy, C₁-C₆ haloalkoxy, C₁-C₆ alkylthio, C₁-C₆ alkylamino, and C₃-C₆ cycloalkyl;
R₅ is independently selected from H, D, C₁-C₆ alkyl, C₃-C₆ cycloalkyl, C₁-C₆ haloalkyl, C₁-C₆ alkoxy, C₃-C₆ cycloalkoxy, and C₁-C₆ haloalkoxy;
R₆ is independently selected from H, D, C₁-C₆ alkyl, C₃-C₆ cycloalkyl, C₁-C₆ haloalkyl, C₁-C₆ alkoxy, and C₁-C₆ haloalkoxy.

In some embodiments of the present invention, R₁ is selected from H, D, methyl, ethyl, *n*-propyl, isopropyl, *n*-butyl, isobutyl, *s*-butyl, *t*-butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, 2-ethylbutyl, cyclopropyl, cyclobutyl, cyclopentyl, cyclohexyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, and pentachloroethyl.

In some embodiments of the present invention, R₂ is independently preferably selected from H, D, and the following groups substituted with R₂ₐ-O-: methyl, ethyl, *n*-propyl, isopropyl, *n*-butyl, isobutyl, *s-*butyl, *t*-butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, and 2-ethylbutyl; R₂ₐ is independently preferably selected from fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, and pentachloroethyl.

In some embodiments of the present invention, R₂ is independently further preferably selected from the following groups substituted with R₂ₐ-O-: methyl, ethyl, *n*-propyl, *n*-butyl, *n*-pentyl, and *n*-hexyl. R₂ₐ is independently further preferably selected from fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, and pentachloroethyl.

In some embodiments of the present invention, R₂ is independently further preferably selected from trifluoromethyl-O-methyl, trifluoromethyl-O-ethyl, trifluoromethyl-O-*n*-propyl, trifluoromethyl-O-*n-*butyl, trifluoromethyl-O-*n*-pentyl, trifluoromethyl-O-*n*-hexyl, trichloromethyl-O-methyl, trichloromethyl-O-ethyl, trichloromethyl-O-*n*-propyl, trichloromethyl-O-*n*-butyl, trichloromethyl-O*n*-pentyl, and trichloromethyl-O-*n*-hexyl.

In some embodiments of the present invention, R₃ₐ, R_{3b}, R_{3c}, R_{3d}, and R₃ₑ are each independently selected from H, D, F, Cl, Br, I, methyl, ethyl, *n*-propyl, isopropyl, *n*-butyl, isobutyl, *s*-butyl, *t*-butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, cyclopropyl, cyclobutyl, cyclopentyl, and cyclohexyl.

In some embodiments of the present invention, R₄ is independently selected from H, D, F, Cl, Br, I, - CN, -NO₂, -NH₂, -OH, -SH, -COOH, methyl, ethyl, *n*-propyl, isopropyl, *n*-butyl, isobutyl, *s*-butyl, *t-*butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, *n*-propoxy, isopropoxy, *n*-butoxy, *s*-butoxy, *t*-butoxy, *n*-pentyloxy, *S*-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, pentachloroethoxy, methylthio, ethylthio, *n*-propylthio, isopropylthio, *n*-butylthio, *s*-butylthio, *t*-butylthio, *n*-pentylthio, *S*-pentylthio, hexylthio, 2-ethylbutylthio, methylamino, dimethylamino, ethylamino, diethylamino, *n*-propylamino, isopropylamino, *n*-butylamino, *s*-butylamino, *t*-butylamino, *n*-pentylamino, *S*-pentylamino, hexylamino, 2-ethylbutylamino, cyclopropyl, cyclobutyl, cyclopentyl, and cyclohexyl.

In some embodiments of the present invention, R₅ is independently selected from H, D, methyl, ethyl, *n*-propyl, isopropyl, *n*-butyl, isobutyl, *s*-butyl, *t*-butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, 2-ethylbutyl, cyclopropyl, cyclobutyl, cyclopentyl, cyclohexyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, *n*-propoxy, isopropoxy, *n*-butoxy, *s*-butoxy, *t*-butoxy, *n*-pentyloxy, *S*-pentyloxy, hexyloxy, 2-ethylbutoxy, cyclopropyloxy, cyclobutoxy, cyclopentyloxy, cyclohexyloxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, and pentachloroethoxy.

In some embodiments of the present invention, R₆ is independently selected from methyl, ethyl, *n-*propyl, isopropyl, *n*-butyl, isobutyl, *s*-butyl, *t*-butyl, *n*-pentyl, isopentyl, neopentyl, 1-ethylpropyl, *n-*hexyl, isohexyl, 1,1-dimethylbutyl, 2,2-dimethylbutyl, 3,3-dimethylbutyl, 2-ethylbutyl, cyclopropyl, cyclobutyl, cyclopentyl, cyclohexyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, *n*-propoxy, isopropoxy, *n*-butoxy, *s*-butoxy, *t*-butoxy, *n*-pentyloxy, *S*-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, and pentachloroethoxy.

In some embodiments of the present invention, provided is a compound of formula (II), a pharmaceutically acceptable salt thereof or a stereoisomer thereof, 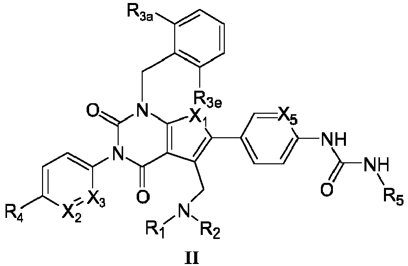 wherein:
X₁ is independently selected from O or S;
X₂ and X₃ are each independently selected from CR₄ or N;
X₅ is independently selected from CH or N;
R₁ is independently selected from H, D, and C₁-C₆ alkyl;
R₂ is independently selected from H, D, C₁-C₆ alkyl, and C₁-C₆ alkyl substituted with R₂ₐ-O-;
R₂ₐ is independently selected from C₁-C₆ haloalkyl;
R₃ₐ and R₃ₑ are each independently selected from H, D, halogen, and C₁-C₆ haloalkyl;
R₄ is independently selected from H, D, halogen, C₁-C₆ alkoxy, C₁-C₆ haloalkoxy, C₁-C₆ alkylthio, and C₁-C₆ alkylamino;
R₅ is independently selected from H, D, C₁-C₆ alkyl, and C₁-C₆ alkoxy.

In some embodiments of the present invention, provided is a compound of formula (II), a pharmaceutically acceptable salt thereof or a stereoisomer thereof,
wherein:
X₁ is independently selected from O or S;
X₂ and X₃ are both selected from N;
X₅ is independently selected from CH or N;
R₁ is independently selected from H, D, and C₁-C₆ alkyl; the C₁-C₆ alkyl is preferably selected from methyl and ethyl;
R₂ is independently selected from H, D, C₁-C₆ alkyl, and C₁-C₆ alkyl substituted with R₂ₐ-O-; the C₁-C₆ alkyl is preferably selected from methyl, ethyl, *n*-propyl, and *n*-butyl;
R₂ₐ is independently selected from C₁-C₆ haloalkyl; the halogen is preferably selected from fluorine and chlorine; the C₁-C₆ haloalkyl is preferably selected from fluoromethyl, difluoromethyl, trifluoromethyl, chloromethyl, dichloromethyl, and trichloromethyl;
R₃ₐ and R₃ₑ are each independently selected from H, D, halogen, and C₁-C₆ haloalkyl; the halogen is preferably selected from fluorine and chlorine; the C₁-C₆ haloalkyl is preferably selected from fluoromethyl, difluoromethyl, trifluoromethyl, chloromethyl, dichloromethyl, and trichloromethyl; R₄ is independently selected from C₁-C₆ alkoxy, C₁-C₆ haloalkoxy, C₁-C₆ alkylthio, and C₁-C₆ alkylamino; the C₁-C₆ alkoxy is preferably selected from methoxy, ethoxy, *n*-propoxy, and *n*-butoxy; the C₁-C₆ haloalkoxy is preferably selected from fluoromethoxy, chloromethoxy, difluoromethoxy, dichloromethoxy, trifluoromethoxy, and trichloromethoxy; the C₁-C₆ alkylthio is preferably selected from methylthio, ethylthio, *n*-propylthio, and *n*-butylthio; the C₁-C₆ alkylamino is preferably selected from methylamino, ethylamino, *n*-propylamino, and *n*-butylamino;
R₅ is independently selected from H, D, C₁-C₆ alkyl, and C₁-C₆ alkoxy; the C₁-C₆ alkyl is preferably selected from methyl, ethyl, *n*-propyl, and *n*-butyl; the C₁-C₆ alkoxy is preferably selected from methoxy, ethoxy, *n*-propoxy, and *n*-butoxy.

In some embodiments of the present invention, provided is a compound of formula (II), a pharmaceutically acceptable salt thereof or a stereoisomer thereof,
wherein:
X₁ is independently selected from O or S;
X₂ and X₃ are both selected from N;
X₅ is independently selected from CH or N;
R₁ is independently selected from H, D, and C₁-C₆ alkyl;
R₂ is C₁-C₆ alkyl substituted with R₂ₐ-O-; preferably, R₂ is independently selected from methyl, ethyl, and propyl, and R₂ₐ is trifluoromethyl;
R₃ₐ and R₃ₑ are each independently selected from H, D, halogen, and C₁-C₆ haloalkyl; the halogen is preferably selected from fluorine and chlorine; the C₁-C₆ haloalkyl is preferably selected from fluoromethyl, difluoromethyl, trifluoromethyl, chloromethyl, dichloromethyl, and trichloromethyl; R₄ is independently selected from C₁-C₆ alkoxy, C₁-C₆ haloalkoxy, C₁-C₆ alkylthio, and C₁-C₆ alkylamino; the C₁-C₆ alkoxy is preferably selected from methoxy, ethoxy, *n*-propoxy, and *n*-butoxy; the C₁-C₆ haloalkoxy is preferably selected from fluoromethoxy, chloromethoxy, difluoromethoxy, dichloromethoxy, trifluoromethoxy, and trichloromethoxy; the C₁-C₆ alkylthio is preferably selected from methylthio, ethylthio, *n*-propylthio, and *n*-butylthio; the C₁-C₆ alkylamino is preferably selected from methylamino, ethylamino, *n*-propylamino, and *n*-butylamino;
R₅ is independently selected from H, D, C₁-C₆ alkyl, and C₁-C₆ alkoxy; the C₁-C₆ alkyl is preferably selected from methyl, ethyl, *n*-propyl, and *n*-butyl; the C₁-C₆ alkoxy is preferably selected from methoxy, ethoxy, *n*-propoxy, and *n*-butoxy.

In some embodiments of the present invention, provided is a compound of formula (II), a pharmaceutically acceptable salt thereof or a stereoisomer thereof,
wherein:
X₁ is independently selected from O or S;
X₂ and X₃ are both selected from CR₄;
X₅ is independently selected from CH or N;
R₁ is independently selected from H, D, and C₁-C₆ alkyl; the C₁-C₆ alkyl is preferably selected from methyl and ethyl;
R₂ is independently selected from H, D, C₁-C₆ alkyl, and C₁-C₆ alkyl substituted with R₂ₐ-O-; the C₁-C₆ alkyl is preferably selected from methyl, ethyl, *n*-propyl, and *n*-butyl;
R₂ₐ is independently selected from C₁-C₆ haloalkyl; the halogen is preferably selected from fluorine and chlorine; the C₁-C₆ haloalkyl is preferably selected from fluoromethyl, difluoromethyl, trifluoromethyl, chloromethyl, dichloromethyl, and trichloromethyl;
R₃ₐ and R₃ₑ are each independently selected from H, D, halogen, and C₁-C₆ haloalkyl; the halogen is preferably selected from fluorine and chlorine; the C₁-C₆ haloalkyl is preferably selected from fluoromethyl, difluoromethyl, trifluoromethyl, chloromethyl, dichloromethyl, and trichloromethyl; R₄ is independently selected from H, D, halogen, C₁-C₆ alkoxy, and C₁-C₆ haloalkoxy; the C₁-C₆ alkoxy is preferably selected from methoxy, ethoxy, n-propoxy, and n-butoxy; the C₁-C₆ haloalkoxy is preferably selected from fluoromethoxy, chloromethoxy, difluoromethoxy, dichloromethoxy, trifluoromethoxy, and trichloromethoxy; the C₁-C₆ alkylthio is preferably selected from methylthio, ethylthio, *n*-propylthio, and *n*-butylthio; the C₁-C₆ alkylamino is preferably selected from methylamino, ethylamino, *n*-propylamino, and *n*-butylamino.
R₅ is independently selected from H, D, C₁-C₆ alkyl, and C₁-C₆ alkoxy; the C₁-C₆ alkyl is preferably selected from methyl, ethyl, *n*-propyl, and *n*-butyl; the C₁-C₆ alkoxy is preferably selected from methoxy, ethoxy, *n*-propoxy, and *n*-butoxy.

In some embodiments of the present invention, provided is a compound of formula (II), a pharmaceutically acceptable salt thereof or a stereoisomer thereof,
wherein:
X₁ is independently selected from O or S;
X₂ and X₃ are both selected from CR₄;
X₅ is independently selected from CH or N;
R₁ is independently selected from H, D, and C₁-C₆ alkyl; the C₁-C₆ alkyl is preferably selected from methyl and ethyl;
R₂ is independently selected from H, D, C₁-C₆ alkyl, and C₁-C₆ alkyl substituted with R₂ₐ-O-; the C₁-C₆ alkyl is preferably selected from methyl, ethyl, *n*-propyl, and *n*-butyl;
R₂ₐ is independently selected from C₁-C₆ haloalkyl; the halogen is preferably selected from fluorine and chlorine; the C₁-C₆ haloalkyl is preferably selected from fluoromethyl, difluoromethyl, trifluoromethyl, chloromethyl, dichloromethyl, and trichloromethyl;
R₃ₐ and R₃ₑ are each independently selected from H, D, halogen, and C₁-C₆ haloalkyl; the halogen is preferably selected from fluorine and chlorine; the C₁-C₆ haloalkyl is preferably selected from fluoromethyl, difluoromethyl, trifluoromethyl, chloromethyl, dichloromethyl, and trichloromethyl; R₄ is C₁-C₆ haloalkoxy, preferably fluoromethoxy, chloromethoxy, difluoromethoxy, dichloromethoxy, trifluoromethoxy, or trichloromethoxy;
R₅ is independently selected from H, D, C₁-C₆ alkyl, and C₁-C₆ alkoxy; the C₁-C₆ alkyl is preferably selected from methyl, ethyl, *n*-propyl, and *n*-butyl; the C₁-C₆ alkoxy is preferably selected from methoxy, ethoxy, *n*-propoxy, and *n*-butoxy.

In some embodiments of the present invention, provided is a compound of formula (III), a pharmaceutically acceptable salt thereof or a stereoisomer thereof, 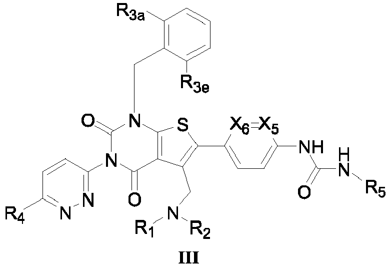 wherein,
X₅ and X₆ are independently selected from CR₆ or N, and X₅ and X₆ are not both CR₆ or N;
R₁ is independently selected from H, D, C₁-C₆ alkyl, C₃-C₆ cycloalkyl, and C₁-C₆ haloalkyl;
R₂ is independently selected from H, D, C₁-C₆ alkyl, and C₁-C₆ alkyl substituted with R₂ₐ-O-;
R₂ₐ is independently selected from C₁-C₆ haloalkyl;
R₃ₐ and R₃ₑ are each independently selected from H, D, halogen, C₁-C₆ alkyl, C₁-C₆ haloalkyl, and C₃-C₆ cycloalkyl;
R₄ is independently selected from H, D, halogen, -CN, -NO₂, -NH₂, -OH, -SH, -COOH, C₁-C₆ alkyl, C₁-C₆ haloalkyl, C₁-C₆ alkoxy, C₁-C₆ haloalkoxy, C₁-C₆ alkylthio, C₁-C₆ alkylamino, and C₃-C₆ cycloalkyl;
R₅ is independently selected from H, D, C₁-C₆ alkyl, C₃-C₆ cycloalkyl, C₁-C₆ haloalkyl, C₁-C₆ alkoxy, C₃-C₆ cycloalkoxy, and C₁-C₆ haloalkoxy.

In some embodiments of the present invention, provided is a compound as shown below, a pharmaceutically acceptable salt thereof or a stereoisomer thereof:

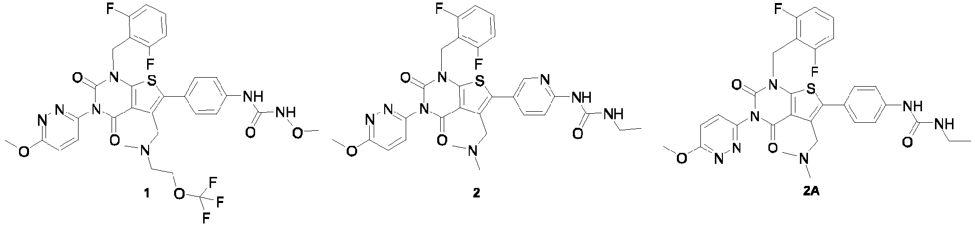

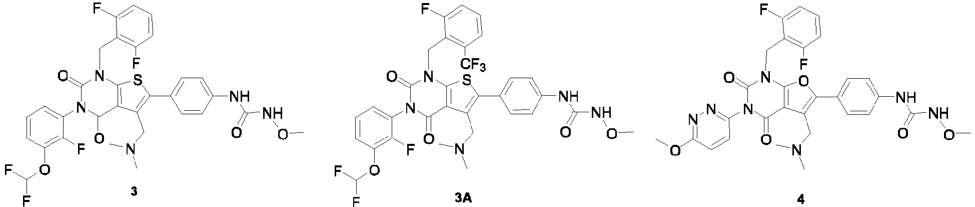

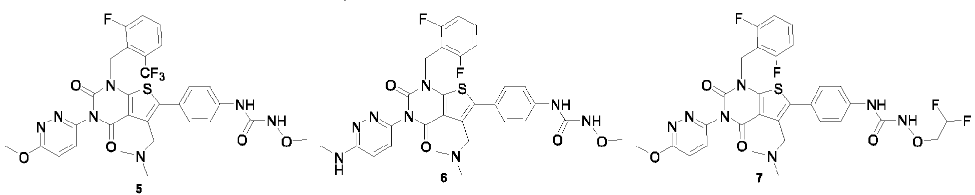

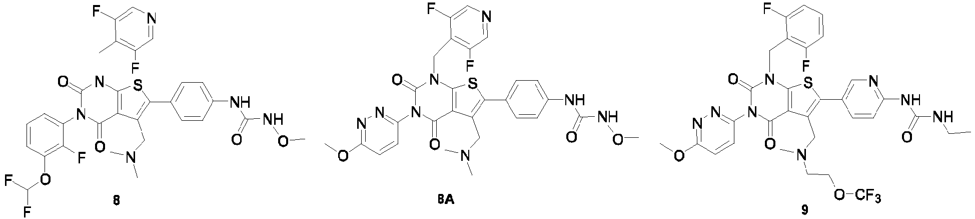

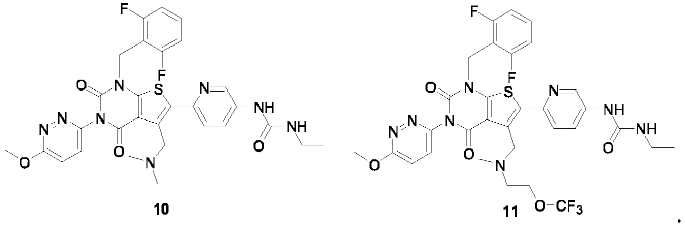

The present invention also provides a pharmaceutical composition, which comprises any one of the compounds or pharmaceutically acceptable salts thereof described above and a pharmaceutically acceptable carrier. The pharmaceutical composition can be prepared into various pharmaceutically acceptable dosage forms, such as tablets, capsules, oral liquid, granules, injections, or various sustained and controlled release preparations. The pharmaceutical composition can be administered orally or parenterally (e.g., intravenously, subcutaneously, topically, etc.). The administration dose may be suitably adjusted depending on the age, sex, and type of disease of the patient, and is generally about 1-200 mg per day.

The present invention also provides use of the compound, the pharmaceutically acceptable salt thereof or the stereoisomer thereof, or the pharmaceutical composition thereof described above in preparing a medicament for preventing or treating a sex hormone-dependent disease. The sex hormone-dependent disease includes sex hormone-dependent cancer, bone metastases of sex hormone-dependent cancer, prostatic hypertrophy, prostatic cancer, hysteromyoma, endometriosis, metrofibroma, precocious puberty, amenorrhea, premenstrual syndrome, dysmenorrhoea, multilocular ovarian syndrome, polycystic ovary syndrome, acne, alopecia, Alzheimer's disease, infertility, irritable bowel syndrome, benign or malignant tumors that are hormone-independent and sensitive to LH-RH (luteinizing hormone-releasing hormone), or flushing. The sex hormone-dependent cancer is selected from prostate cancer, uterine cancer, breast cancer, and pituitary cancer.

In some embodiments of the present invention, provided is use of the compound, or the pharmaceutically acceptable salt thereof or the stereoisomer thereof, or the pharmaceutical composition thereof described above in preparing a reproduction modulator, a contraceptive, or an ovulation inducer; or use thereof as a medicament for the prevention of postoperative recurrence of sex hormone-dependent cancer.

The compounds provided by the present invention have one or more of the following technical advantages:
1. having significant binding ability to human GnRHR;
2. having a significant inhibitory effect on human GnRHR;
3. having lower inhibitory activity on the human hERG potassium channel, and having lower cardiac toxicity;
4. being capable of improving the permeability in Caco-2 cells;
5. being capable of improving the exposure and the absolute bioavailability of the drug *in vivo*, and having excellent pharmacokinetic properties;
6. having a higher drug concentration in the pituitary of the target organ.

### DEFINITION AND DESCRIPTION

Unless otherwise stated, the following terms and phrases used herein are intended to have the following meanings. A specific term or phrase, unless otherwise specifically defined, should not be considered uncertain or unclear, but construed according to its ordinary meaning. When referring to a trade name, it is intended to refer to its corresponding commercial product or its active ingredient.

The term "pharmaceutically acceptable" is described herein for the compounds, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" described herein refers to a salt of the compound of the present invention, which is prepared from the compound having particular substituents of the present invention and a relatively nontoxic acid or base. When the compound of the present invention contains a relatively basic functional group, an acid addition salt can be obtained by contacting the neutral form of such a compound with a sufficient amount of an acid in a pure solution or a suitable inert solvent. Examples of the pharmaceutically acceptable acid addition salts include inorganic acid salts and organic acid salts, and also include salts of amino acids such as arginine, and salts of organic acids such as glucuronic acid. Certain particular compounds of the present invention contain basic functional group that allow the compounds to be converted into any acid addition salt.

Certain compounds of the present invention may have asymmetric carbon atoms (optical centers) or double bonds. Racemates, diastereoisomers, geometric isomers, and individual isomers are all included within the scope of the present invention.

The compounds of the present invention can be in the form of a particular geometric isomer or stereoisomer. All such compounds are contemplated herein, including cis and trans isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present invention. Substituents such as alkyl may have an additional asymmetric carbon atom. All these isomers and mixtures thereof are encompassed within the scope of the present invention.

The pharmaceutically acceptable salt of the present invention can be synthesized from the parent compound containing an acid radical or a basic group by conventional chemical methods. In general, the salt is prepared by reacting the compounds in the form of a free acid or base with a stoichiometrically appropriate base or acid in water or an organic solvent or a mixture of the two.

The term "pharmaceutically acceptable carrier" refers to any preparation or carrier medium representative of a carrier that is capable of delivering an effective amount of an active substance of the present invention, does not interfere with the biological activity of the active substance, and has no toxic side effects on the host or patient, including, but not limited to: binders, fillers, lubricants, disintegrants, wetting agents, dispersing agents, solubilizers, suspending agents, and the like.

The present invention is intended to include all isotopes of atoms present in the compounds of the present invention. Isotopes include those atoms having the same number of atoms but different mass numbers. As a general example and not by way of limitation, isotopes of hydrogen include deuterium and tritium. Isotopes of carbon include ¹³C and ¹⁴C. Isotopically-labeled compounds of the present invention can generally be prepared by conventional techniques known to those skilled in the art or by processes analogous to those described herein using an appropriate isotopically-labeled reagent in place of the non-labeled reagent otherwise used.

Unless otherwise specified, the term "alkyl" is intended to refer to linear or branched saturated hydrocarbyl, which may be monosubstituted (e.g., -CH₂F) or polysubstituted (e.g., -CF₃), and may be monovalent (e.g., methyl), divalent (e.g., methylene), or multivalent (e.g., methine). For example, C₁-C₆ represents 1-6 carbons, C₁₋₁₀ is selected from C₁, C₂, C₃, C₄, C₅, and C₆. Examples of the alkyl include methyl (Me), ethyl (Et), propyl (e.g., *n*-propyl and isopropyl), butyl (e.g., *n*-butyl, isobutyl, *s-*butyl, and *t*-butyl), pentyl (e.g., *n*-pentyl, isopentyl, neopentyl, and 1-ethylpropyl), hexyl (e.g., *n*-hexyl, isohexyl, 1,1-dimethylbutyl, 2,2-dimethylbutyl, 3,3-dimethylbutyl, and 2-ethylbutyl), and the like. Unless otherwise specified, the term "halo" or "halogen" by itself or as part of another substituent refers to a fluorine, chlorine, bromine, or iodine atom.

"Haloalkyl" is intended to include monohalogenated alkyl and polyhalogenated linear or branched alkyl. For example, the term "C₁-C₆ haloalkyl" is intended to include, but not be limited to, trifluoromethyl, 2,2,2-trifluoroethyl, 4-chlorobutyl, and 3-bromopropyl. Unless otherwise specified, examples of C₁-C₆ haloalkyl include, but are not limited to: fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, and pentachloroethyl.

Unless otherwise specified, "alkoxy" represents the above alkyl groups (including cycloalkyl or haloalkyl) having the specified number of carbon atoms connected through an oxygen bridge. Typical alkoxy groups include C₁₋₆ alkoxy, e.g., alkoxy of C₁, C₂, C₃, C₄, C₅, and C₆, cycloalkoxy of C₃, C₄, C₅, and C₆, and haloalkoxy of C₁, C₂, C₃, C₄, C₅, and C₆. Examples of the alkoxy include, but are not limited to: methoxy, ethoxy, *n*-propoxy, isopropoxy, *n*-butoxy, *s*-butoxy, *t*-butoxy, *n*-pentyloxy, *S-*pentyloxy, hexyloxy, and 2-ethylbutoxy. Examples of the cycloalkoxy include, but are not limited to: cyclopropoxy, cyclobutoxy, cyclopentyloxy, and cyclohexyloxy. Examples of haloalkoxy include, but are not limited to: fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, and pentachloroethoxy.

Unless otherwise specified, the term "alkylthio" refers to an alkyl group attached to the rest of the molecule through a sulfur atom, wherein the alkyl group has the meaning as described herein. In some embodiments, the alkylthio group contains 1-6 carbon atoms; in some other embodiments, the alkylthio group contains 1-4 carbon atoms; in still some other embodiments, the alkylthio group contains 1-3 carbon atoms. The alkylthio group can be optionally substituted with one or more substituents described herein. Examples of the alkylthio group include, but are not limited to, methylthio (MeS or -SCH₃), ethylthio (EtS or -SCH₂CH₃), *n*-propylthio, isopropylthio, *n*-butylthio, *s*-butylthio, *t*-butylthio, *n*-pentylthio, *S*-pentylthio, hexylthio, 2-ethylbutylthio, and the like.

Unless otherwise specified, the term "alkylamino" or "alkylamino" means that the above amino group is independently substituted with one or two alkyl groups, including "*N*-alkylamino" and "*N*,*N-*dialkylamino", wherein the alkyl groups have the meaning as described herein. In some embodiments, a suitable alkylamino group can be monoalkylamino or dialkylamino, in which each alkyl contains 1-6 carbon atoms. Examples of such alkylamino groups include, but are not limited to, *N*-methylamino (methylamino), *N*-ethylamino (ethylamino), *N*,*N*-dimethylamino (dimethylamino), *N*,*N*-diethylamino (diethylamino), *n*-propylamino, isopropylamino, *n*-butylamino, *s*-butylamino, *t*-butylamino, *n-*pentylamino, *S*-pentylamino, hexylamino, 2-ethylbutylamino, and the like.

Unless otherwise specified, cycloalkyl includes any stable cyclic or polycyclic hydrocarbon group with any carbon atom being saturated, which may be monosubstituted or polysubstituted and may be monovalent, divalent, or polyvalent. Examples of such cycloalkyl include, but are not limited to, cyclopropyl, cyclobutyl, cyclopentyl, cyclohexyl, and the like.

Compounds are named either manually or by ChemDraw^{®} software, and supplier's catalog names are given for commercially available compounds.

### BRIEF DESCRIPTION OF THE DRAWINGS

FIG. 1: average concentration-time curve after intravenous (1 mg/kg) administration in SD rats
FIG. 2: average concentration-time curve after intragastric (12 mg/kg) administration in SD rats

### DETAILED DESCRIPTION

The present invention will be further illustrated with reference to specific examples and test examples, but the scope of the present invention is not limited in any way.

### Example 1. Synthesis of Compound 1

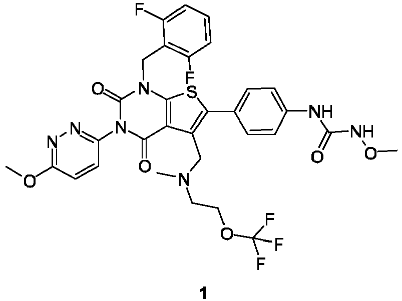

### Synthetic route:

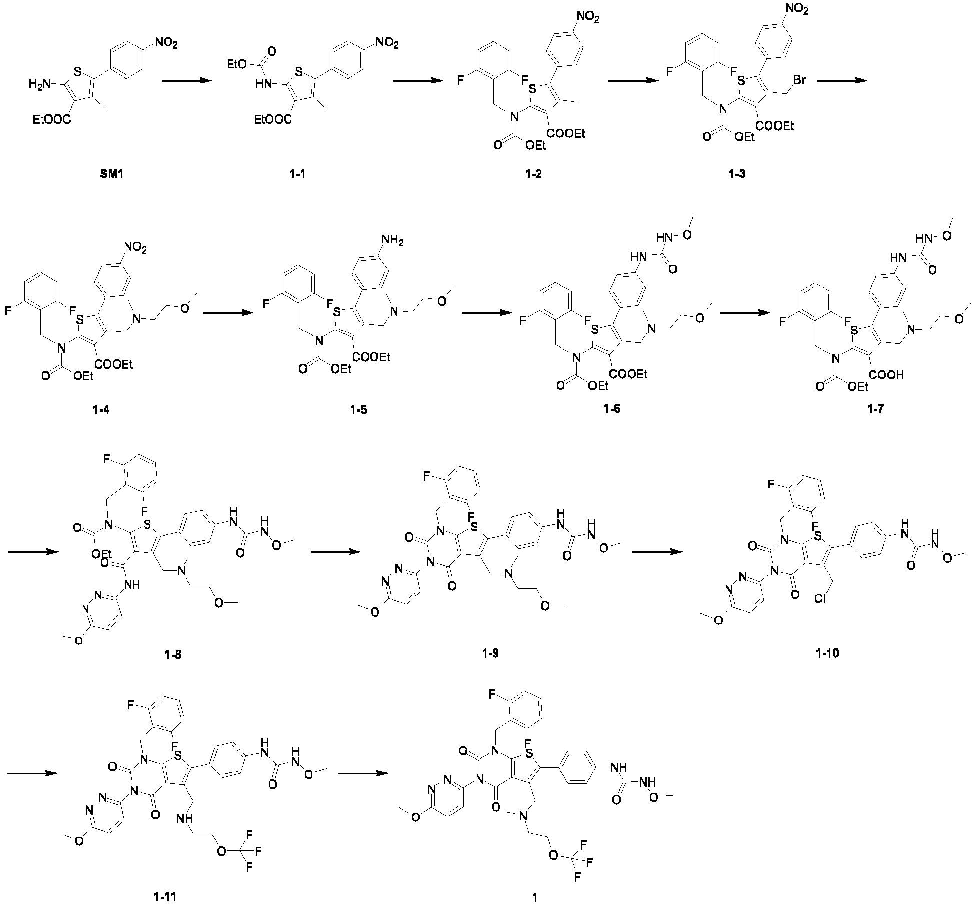

### Step 1: synthesis of compound 1-1

Ethyl chloroformate (56.2 mL, 588 mmol) was added to compound SM1 (90.0 g, 294 mmol) in toluene (2.36 L), and the mixture was reacted at 110 °C for 3 h. Ethyl chloroformate (56.2 mL, 588 mmol) was added again, and the mixture was reacted at 110 °C for 3 h. After that, ethyl chloroformate (56.2 mL, 588 mmol) was additionally added, and the mixture was reacted at 110 °C for another 6 h, cooled to 25 °C, and concentrated under vacuum. To the residue was added 600 mL of methyl *t*-butyl ether. The filter cake was collected by filtration, washed with 100 mL of methyl *t*-butyl ether, and dried to give compound 1-1 (107.57 g, 96.8%) as a yellow powder. MS m/z (ESI): 379[M+H]⁺; ¹H NMR (400 MHz CDCl₃), δ 1.36 (t, *J* = 7.20 Hz, 3H), 1.42 (t, *J* = 7.20 Hz, 3H), 2.42 (s, 3 H), 4.31 (q, *J* = 7.20 Hz, 2 H), 4.40 (q, *J* = 7.20 Hz, 2H), 7.53 - 7.59 (m, 2 H), 8.24 - 8.29 (m, 2 H), 10.66 (s, 1 H).

### Step 2: synthesis of compound 1-2

To a solution of compound 1-1 (107.57 g, 284.28 mmol, 1 eq) in DMF (3400 mL) were added K₂CO₃ (43.22 g, 312.71 mmol, 1.1 eq) and KI (51.91 g, 312.71 mmol, 1.1 eq). Then, to the mixture was added a solution of 2,6-difluorobenzyl chloride (55.46 g, 341.14 mmol, 1.2 eq) in DMF (190 mL). The mixture was stirred at 25 °C for 17 h. The precipitate thus produced was filtered off and the filtrate was concentrated under vacuum. The residue was diluted with ethyl acetate (700 mL) and water (700 mL), and extracted with ethyl acetate (300 mL × 2). The extract was washed with 500 mL of water and 500 mL of brine, dried over anhydrous Na₂SO₄, and concentrated under reduced pressure. The residue was stirred in MTBE (540 mL) at 25 °C for 30 min. The precipitate was collected by filtration to give compound 1-2 (127 g, 251.73 mmol, yield: 88.55%) as a pale yellow solid. MS m/z (ESI): 505[M+H]⁺; ¹H NMR (400 MHz CDCl₃), δ1.20 (s, 2H), 1.33 (t, *J* = 6.80 Hz, 4H), 2.40 (s, 3H), 4.18 - 4.34 (m, 4H), 4.98 (s, 2H), 6.86 (t, *J* = 8.00 Hz, 2H), 7.22 - 7.31 (m, 1H), 7.52 (d, *J* = 8.80 Hz, 2H), 8.25 - 8.30 (m, 2H).

### Step 3: synthesis of compound 1-3

Compound 1-2 (50 g, 99.11 mmol, 1 eq), NBS (22.05 g, 123.88 mmol, 1.25 eq), and AIBN (1.63 g, 9.91 mmol, 0.1 eq) were stirred in ethyl acetate (600 mL) at 80 °C for 2 h. NBS (3.53 g, 19.82 mmol, 0.20 eq) and AIBN (162.74 mg, 991.08 µmol, 0.01 eq) were additionally added to the system. The resulting mixture was heated at 80 °C for 2 h. After cooling to room temperature, the reaction mixture was diluted with a diluent (200 mL) and water (300 mL) and extracted (200 mL × 2). The extract was washed with 300 mL of water and 500 mL of brine, dried over Na₂SO₄, and concentrated under reduced pressure to give a crude product of compound 1-3 (crude, 61.55 g), which was used in the next step. MS m/z (ESI): 585,583[M+H]⁺.

### Step 4: synthesis of compound 1-4

To a solution of compound 1-3 (40.0 g, 68.5 mmol) in ethyl acetate (400 mL) were added DIPEA (17.7 g, 137 mmol, 23.9 mL) and *N*-(2-methoxyethyl)methylamine (9.17 g, 103 mmol, 11.0 mL), and the mixture was stirred at 25 °C for 16 h. After LCMS showed that the starting material had been completely consumed, the mixture was washed with water (200 mL × 2) and brine (150 mL) and extracted with ethyl acetate (200 mL) to separate the aqueous layer. The organic layers were combined, dried over Na₂SO₄, and concentrated under reduced pressure to remove the solvent. The residue was purified by silica gel column chromatography (CH₂Cl₂:MeOH = 10:1) to give compound 1-4 (60.0 g, 95.3%) as a yellow oil. MS m/z (ESI): 592[M+H]⁺; ¹H NMR (400 MHz CDCl₃), δ8.24 (d, *J* = 8.0 Hz, 2H), 7.70 (d, *J* = 8.4 Hz, 2H), 7.29 - 7.25 (m, 1H), 6.86 (t, *J* = 8.0 Hz, 2H), 5.01 (s, 2H), 4.23 - 4.20 (m, 4H), 3.63 (s, 2H), 3.37 - 3.36 (m, 2H), 3.28 (s, 3H), 2.46 (s, 2H), 2.08 (s, 3H), 1.35 - 1.19 (m, 6H).

### Step 5: synthesis of compound 1-5

Compound 1-4 (60.0 g, 101 mmol) was added to an EtOH (900 mL) solution, and Pd/C (12.0 g, 20.28 mmol, purity: 10%, 0.2 eq) was added under N₂ atmosphere. Then the mixture was degassed under vacuum and replaced 3 times with H₂. The mixture was stirred under H₂ atmosphere (25 psi) at 25 °C for 16 h. After TLC (CH₂Cl₂:MeOH = 10:1, Rf = 0.2) showed that the starting material had been completely consumed, the mixture was filtered, and the filter cake was washed with EtOH (500 mL) and THF (500 mL). The mixture was concentrated under reduced pressure to remove the organic layer. The residue was dissolved in tetrahydrofuran (150 mL), and the resulting mixture was concentrated under reduced pressure to remove the solvent. This process was repeated three times to give compound 1-5 (56.0 g, 90.5 mmol, yield: 89.2%, purity: 90.8%) as a brown oil. MS m/z (ESI): 562[M+H]⁺; ¹H NMR (400 MHz CDCl₃), δ7.25 - 7.21 (m, 1H), 7.17 (d, *J* = 6.8 Hz, 2H), 6.83 (t, *J* = 8.0 Hz, 2H), 6.66 (d, *J* = 8.0 Hz, 2H), 4.99 (s, 2H), 4.23 - 4.18 (m, 4H), 3.59 (s, 2H), 3.34 - 3.31 (m, 2H), 3.26 (s, 3H), 2.39 (s, 2H), 2.05 (s, 3H), 1.32 - 1.18 (m, 6H).

### Step 6: synthesis of compound 1-6

To a solution of compound 1-5 (56.0 g, 99.7 mmol) in dichloromethane (600 mL) were added DIPEA (25.7 g, 199 mmol, 34.7 mL) and CDI (32.3 g, 199 mmol), and the mixture was stirred at 25 °C for 16 h. After the reaction was completed, DIPEA (64.4 g, 498 mmol, 86.8 mL) was added, methoxyamine (41.6 g, 498 mmol, HCl) was added in portions at 0 °C, and the mixture was stirred at 0-25 °C for 4 h. After LCMS showed that the starting material had been completely consumed, the mixture was diluted with CH₂Cl₂ (1.00 L), washed with water (500 mL) and brine (500 mL), dried over Na₂SO₄, and concentrated under reduced pressure to remove the solvent. The residue was purified by silica gel column chromatography to give compound 1-6 (64.0 g, 92.7 mmol, yield: 93.0%, purity: 92.0%) as a yellow oil. MS m/z (ESI): 635[M+H]⁺; ¹H NMR (400 MHz CDCl₃), δ7.64 (br.s, 1H), 7.51 (d, *J* = 8.4 Hz, 2H), 7.37 (d, *J* = 8.4 Hz, 2H), 7.25 - 7.21 (m, 1H), 6.85 (t, *J* = 8.4 Hz, 2H), 5.00 (s, 2H), 4.23 - 4.19 (m, 4H), 3.82 (s, 3H), 3.63 - 3.59 (m, 2H), 3.37 - 3.36 (m, 2H), 3.27 (s, 3H), 2.43 (s, 2H), 2.07 (s, 3H), 1.33 - 1.19 (m, 6H).

### Step 7: synthesis of compound 1-7

To a solution of compound 1-6 (80.0 g, 126 mmol) in ethanol (700 mL) was added NaOH (2 M, 315.11 mL, 5 eq), and the mixture was stirred at 60 °C for 16 h. After LCMS showed that the starting material had been completely consumed, the mixture was concentrated under reduced pressure to remove the solvent and then adjusted to pH 6-7 with aq. HCl. The aqueous layer was extracted with ethyl acetate (1.00 L × 10). The organic layer was combined, dried over Na₂SO₄, and concentrated under reduced pressure to remove the solvent to give compound 1-7 (67.0 g, 96.9 mmol, yield: 76.9%, purity: 87.8%) as a yellow solid. MS m/z (ESI): 607[M+H]⁺; ¹H NMR (400 MHz DMSO-d6), δ9.68 (br.s, 1H), 9.15 (s, 1H), 7.69 (d, *J* = 8.4 Hz, 2H), 7.41 - 7.37 (m, 1H), 7.16 (d, *J* = 8.4 Hz, 2H), 7.04 (t, *J* = 8.0 Hz, 2H), 4.89 (s, 2H), 3.97 - 3.93 (m, 2H), 3.62 (s, 3H), 3.49 - 3.46 (m, 2H), 3.18 (s, 3H), 2.83 (s, 2H), 2.38 (s, 3H), 1.07 (t, *J* = 7.2 Hz, 3H).

### Step 8: synthesis of compound 1-8

A solution of compound 1-7 (60.0 g, 98.9 mmol) and 3-amino-6-methoxypyridazine (25.0 g, 199 mmol) were dissolved in DMF (1800 mL), DIPEA (127 g, 989 mmol, 172 mL) and 1-propylphosphoric anhydride (189 g, 296 mmol, 176 mL, purity: 50%) were added, and then the mixture was mixed with stirring at 25 °C for 3 h. After LCMS showed that the starting material had been completely consumed, the mixture was poured into cold water (4.00 L), and the aqueous layer was extracted with ethyl acetate (2.00 L × 3). The combined organic layer was washed with water (1.00 L) and brine (1.00 L), dried over Na₂SO₄, and concentrated under reduced pressure to remove the solvent to give compound 1-8 (64.0 g, 56.4 mmol, yield: 57.0%, purity: 62.9%) as a brown oil. MS m/z (ESI): 714[M+H]⁺.

### Step 9: synthesis of compound 1-9

A solution of compound 1-8 (64.0 g, 56.4 mmol, purity: 62.9%) in methanol (900 mL) was added in portions to CH₃ONa (30.5 g, 564 mmol), and the mixture was stirred at 25 °C for 16 h. After LCMS showed that the starting material had been completely consumed, the mixture was poured into a cold aq. NH₄Cl solution (1.50 L). The resulting mixture was extracted with ethyl acetate (1.50 L × 2), washed with water (1.00 L) and brine (500 mL), dried over anhydrous Na₂SO₄, and concentrated under reduced pressure to remove the solvent. The residue was purified by silica gel column chromatography to give compound 1-9 (24.0 g, 32.3 mmol, yield: 57.3%, purity: 90.0%) as a yellow solid. MS m/z (ESI): 668[M+H]⁺.

### Step 10: synthesis of compound 1-10

To a solution of compound 1-9 (4.80 g, 7.19 mmol) in tetrahydrofuran (200 mL) was added a solution of 1-chloroethyl chloroformate (1.58 g, 11.0 mmol) in tetrahydrofuran (20 mL), and the mixture was stirred at -70 °C to 25 °C for 3 h. After LCMS showed that the starting material had been completely consumed, the mixture was poured into 1.00 L of water. The resulting mixture was extracted with ethyl acetate (1.00 L × 2). The organic layer was washed with brine (500 mL), dried over Na₂SO₄, and concentrated under reduced pressure to remove the solvent. The residue was slurried with methyl *t-*butyl ether (500 mL), purified, and filtered to give compound 1-10 (22.0 g, 30.7 mmol, yield: 85.6%, purity: 86.0%) as a yellow solid. MS m/z (ESI): 615[M+H]⁺; ¹H NMR (400 MHz CDCl₃), δ7.70 (br.s, 1H), 7.62 (d, *J* = 8.8 Hz, 2H), 7.53 (d, *J* = 8.8 Hz, 2H), 7.45 (d, *J* = 9.2 Hz, 1H), 7.40 - 7.25 (m, 1H), 7.15 (d, *J* = 8.8 Hz, 1H), 6.94 (d, *J* = 8.0 Hz, 2H), 5.35 (s, 2H), 4.80 (s, 2H), 4.20 (s, 3H), 3.83 (s, 3H).

### Step 11: synthesis of compound 1-11

To a solution of compound 1-10 (22.0 g, 35.8 mmol) and 2-(trifluoromethoxy)ethylamine (23.7 g, 143 mmol, HCl) in DMF (500 mL) was added DIPEA (41.6 g, 322 mmol, 56.1 mL), and then the mixture was stirred at 25 °C for 24 h. After the reaction was completed, the mixture was poured into 1.50 L of water. The resulting mixture was extracted with ethyl acetate (1.00 L × 3), washed with water (500 mL × 2), and concentrated under reduced pressure to remove the solvent. The residue was purified by silica gel column chromatography to give compound 1-11 (15.5 g, 19.0 mmol, yield: 53.2%, purity: 87.0%) as a yellow solid. MS m/z (ESI): 708[M+H]⁺; ¹H NMR (400 MHz CDCl₃), δ7.68 (br.s, 1H), 7.58 (d, *J* = 8.4 Hz, 2H), 7.46 (d, *J* = 8.4 Hz, 2H), 7.41 (d, *J* = 9.2 Hz, 1H), 7.35 - 7.27 (m, 2H), 7.16 (d, *J* = 9.2 Hz, 1H), 6.93 (d, *J* = 8.0 Hz, 2H), 5.36 (s, 2H), 4.20 (s, 3H), 4.03 - 4.01 (m, 2H), 3.91 (s, 2H), 3.82 (s, 3H), 3.90 - 3.89 (m, 2H).

### Step 12: synthesis of compound 1

Compound 1-11 (5.00 g, 6.36 mmol, purity: 90.0%) and paraformaldehyde (1.15 g, 12.7 mmol) were dissolved in methanol (150 mL), acetic acid (38.2 mg, 636 µmol, 36.4 µL) and NaBH₃CN (1.60 g, 25.4 mmol) were added, and then the mixture was stirred at 25 °C for 16 h. After LCMS showed that the starting material had been completely consumed, the mixture was poured into an aq. NH₄Cl solution (1500 mL). The resulting mixture was extracted with ethyl acetate (1.00 L × 2), and washed with water (500 mL) and brine (500 mL). The residue was purified by silica gel column chromatography (100% EtAOc) and SFC (column: DAICEL CHIRALCEL OD (250 mm × 30 mm,10 µm), mobile phase: [0.1% NH₃H₂O ETOH], B%: 45%-45%) to give compound 1 (4.98 g, 6.57 mmol, yield: 34.4%, purity: 95.2%) as a white solid. MS m/z (ESI): 722[M+H]⁺; ¹H NMR (400 MHz DMSO-d6), δ9.60 (br.s, 1H), 9.07 (br.s, 1H), 7.74 - 7.70 (m, 3H), 7.57 (d, *J* = 8.8 Hz, 2H), 7.45 (d, *J* = 9.2 Hz, 2H), 7.14 (d, *J* = 8.0 Hz, 2H), 5.37 - 5.22 (m, 2H), 4.09 (s, 3H), 4.03 - 4.00 (m, 2H), 3.80 - 3.68 (m, 2H), 3.63 (s, 3H), 2.65 (s, 2H), 2.09 (s, 3H). ¹⁹F NMR: (400 MHz DMSO-d6), δ-58.744 ppm, -112.961 ppm.

### Example 2. Synthesis of Compound 2

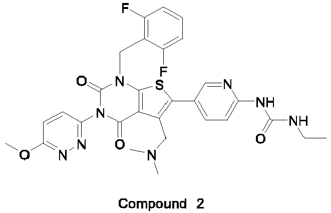

### Synthetic route:

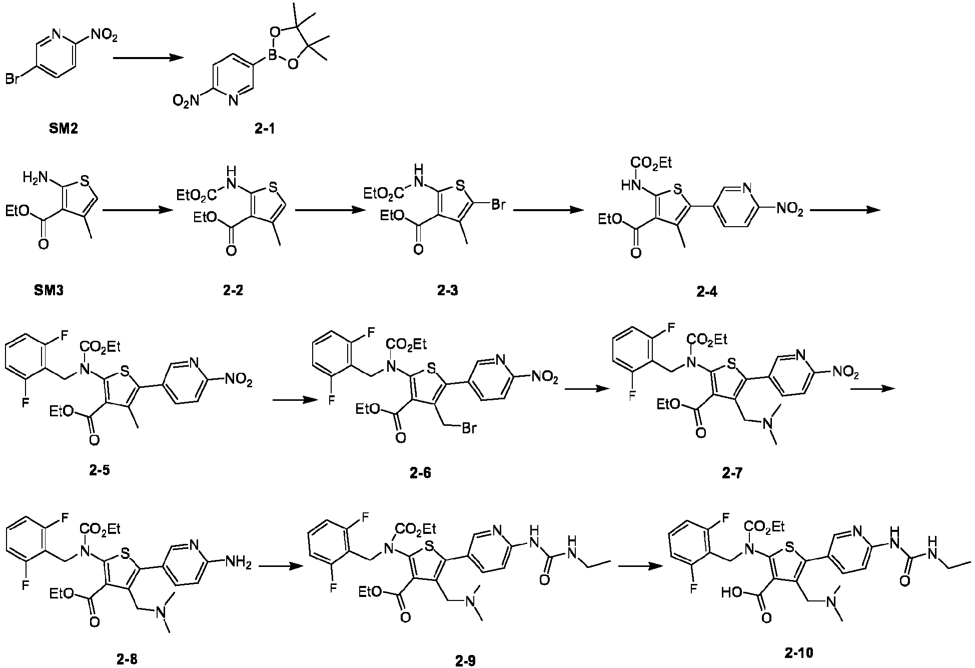

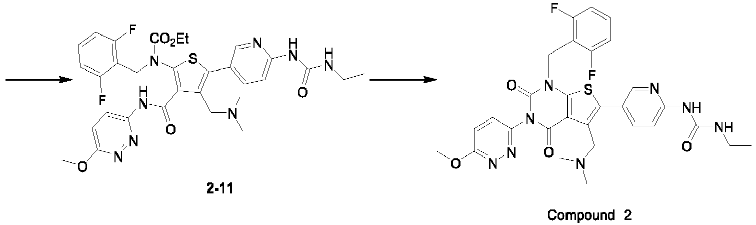

### Synthesis of compound 2-1:

Bis(pinacolato)diboron (75.1 g, 296 mmol, 1.20 eq) and Pd(dppf)Cl₂ (7.21 g, 9.85 mmol, 0.04 eq) were added to a solution of SM2 (50.0 g, 246 mmol, 1.00 eq) and AcOK (120.87 g, 1.23 mol, 5.00 eq) in dioxane (600 mL) under N₂ atmosphere. The mixture was stirred at 100 °C for 1.5 h. After LCMS showed that SM2 had been consumed and a main peak had been detected, the reaction mixture was filtered through celite. The filtrate was concentrated under vacuum to give a residue. The residue was purified by silica gel column chromatography (petroleum ether/ethyl acetate = 75/25) and slurried with petroleum ether/ethyl acetate = 5/1 (420 mL) at 25 °C for 2 h to give compound 2-1 (20 g, 77.5 mmol, yield: 72.0%, purity: 96.9%) as a white solid. MS m/z (ESI): 169[M+H]⁺; ¹H NMR (400 MHz CDCl₃), δppm 8.94 (s, 1 H), 8.38 - 8.40 (m, 1 H), 8.21 (d, *J* = 8.00 Hz, 1 H), 1.37 (s, 12 H).

### Step 1: synthesis of compound 2-2

Ethyl chloroformate (58.9 g, 542 mmol, 51.7 mL, 5.03 eq) was added to a solution of SM3 (20.0 g, 108 mmol, 1.00 eq) in toluene (200 mL). The mixture was stirred at 110 °C for 16 h. After LCMS showed that SM3 had been consumed and a main peak had been detected, the mixture was concentrated under reduced pressure to give a residue, which was directly used in the next step without purification to give compound 2-2 (27.6 g, 97.7 mmol, yield: 90.5%, purity: 91.1%) as a grey solid. MS m/z (ESI): 258[M+H]⁺.

### Step 2: synthesis of compound 2-3

To a solution of compound 2-2 (27.1 g, 105.32 mmol, 1 eq) in CHCl₃ (220 mL) was added NBS (20.6 g, 115.9 mmol, 1.10 eq) at 0 °C. The mixture was stirred at 0 °C for 2 h. After LCMS showed that compound 2-2 had been consumed and a main peak had been detected, the mixture was concentrated under reduced pressure to give a residue. The residue was purified by column chromatography (SiO₂, petroleum ether/ethyl acetate = 94/6) to give compound 2-3 (36.2 g, 93.0 mmol, yield: 71.1%, purity: 86.293%) as a white solid. MS m/z (ESI): 336,338[M+H]⁺; ¹H NMR (400 MHz DMSO-d6), δ ppm 4.20 - 4.29 (m, 4 H), 2.22 (s, 3H), 1.24 - 1.33 (m, 6H).

### Step 3: synthesis of compound 2-4

Compound 2-1 (33.92 g, 135.63 mmol, 2.4 eq), K₂CO₃ (15.62 g, 113.03 mmol, 2 eq), and Pd(dppf)Cl₂ (4.14 g, 5.65 mmol, 0.1 eq) were added to a solution of compound 2-3(19 g, 56.51 mmol, 1 eq) in dioxane (180 mL) and H₂O (18 mL). The mixture was stirred at 80°C for 18 h. After LCMS indicated that compound 2-3 had been consumed and many new peaks had been formed, the mixture was poured into water (100 mL). The resulting mixture was extracted with ethyl acetate (100 mL × 3). The combined organic layer was washed with brine (200 mL), dried over sodium sulfate, filtered, and concentrated under reduced pressure to give a residue. The residue was purified by column chromatography (SiO₂, petroleum ether/dichloromethane = 30/70, Rf = 0.26) to give compound 2-4 (9.48 g, 22.80 mmol, yield: 19.22%, purity: 91.226%) as a yellow solid. MS m/z (ESI): 380[M+H]⁺.

### Step 4: synthesis of compound 2-5

To a solution of compound 2-4 (6.00 g, 15.8 mmol, 1.00 eq), KI (2.89 g, 17.4 mmol, 1.10 eq), and K₂CO₃ (2.40 g, 17.4 mmol, 1.10 eq) in DMF (50 mL) was added 2,6-difluorobenzyl chloride (3.09 g, 19.0 mmol, 1.20 eq), and the mixture was mixed with stirring at 25 °C for 6 h. After LCMS showed that compound 2-4 had been consumed and a major peak had been formed, the mixture was poured into water (100 mL). The resulting mixture was extracted with ethyl acetate (100 mL × 3), washed with brine (200 mL × 1), dried over anhydrous Na₂SO₄, and concentrated under reduced pressure to remove the solvent. The residue was purified by column chromatography (SiO₂, petroleum ether/ethyl acetate = 80/20) to give compound 2-5 (7.26 g, 14.3 mmol, yield: 90.6%, purity: 99.8%) as a yellow oil. MS m/z (ESI): 506[M+H]⁺; ¹H NMR (400 MHz CDCl₃), δ ppm 8.60 (d, *J* = 1.75 Hz, 1 H), 8.30 (d, *J* = 8.38 Hz, 1 H), 7.99 - 8.01 (m, 1 H), 7.24 - 7.30 (m, 1 H), 6.82 - 6.88 (m, 2 H), 4.97 (s, 2 H), 4.08 - 4.27 (m, 4 H), 2.40 (s, 3 H), 1.30 (m, 3 H), 1.18 - 1.25 (m, 3 H).

### Step 5: synthesis of compound 2-6

To a solution of compound 2-5 (7.26 g, 14.4 mmol, 1.00 eq) in ethyl acetate (50 mL) were added NBS (3.07 g, 17.2 mmol, 1.20 eq) and AIBN (235.84 mg, 1.44 mmol, 0.1 eq), and then the mixture was stirred at 80 °C for 16 h. NBS (3.07 g, 17.2 mmol, 1.20 eq) and AIBN (236 mg, 1.44 mmol, 0.10 eq) were added, and the mixture was stirred at 80 °C for 4 h. NBS (3.07 g, 17.2 mmol, 1.20 eq) and AIBN (236 mg, 1.44 mmol, 0.10 eq) were added, and the mixture was stirred at 80 °C for 16 h. After LCMS indicated that compound 2-5 had been consumed and a major peak had been formed, the mixture was diluted with ethyl acetate (100 mL) and washed with brine (50 mL × 2). The aqueous layer was extracted with ethyl acetate (50 mL). The organic layer was dried over Na₂SO₄ and concentrated under reduced pressure to remove the solvent. The residue was directly used in the next step without purification to give compound 2-6 (13 g, crude) as a brown oil. MS m/z (ESI): 584,586[M+H]⁺.

### Step 6: synthesis of compound 2-7

To a solution of compound 2-6 (13.0 g, 22.3 mmol, 1.00 eq) in DMF (100 mL) was added DIEA (7.19 g, 55.6 mmol, 9.69 mL, 2.50 eq), then Me₂NH·HCl (2.39 g, 29.4 mmol, 1.32 eq) was added in portions, and the mixture was stirred at 25 °C for 5 h. After LCMS indicated that compound 2-6 had been consumed and a major peak had been formed, the mixture was poured into water (100 mL). The resulting mixture was extracted with ethyl acetate (150 mL × 3), washed with brine (100 mL × 2), dried over Na₂SO₄, and concentrated under reduced pressure to remove the solvent. The residue was purified by silica gel column chromatography (petroleum ether/ethyl acetate = 60/40) to give compound 2-7 (4.10 g, 6.30 mmol, yield: 28.3%, purity: 84.3%) as a yellow oil. MS m/z (ESI): 549[M+H]⁺; ¹H NMR (400 MHz CDCl₃), δ ppm 8.76 (d, *J* = 1.38 Hz, 1 H), 8.24 - 8.30 (m, 2 H), 7.22 - 7.28 (m, 1 H), 6.81 - 6.86 (m, 2 H), 5.00 (s, 2 H), 4.18 - 4.26 (m, 4 H), 3.53 (s, 2 H), 2.10 (s, 6 H), 1.18 - 1.32 (m, 7H).

### Step 7: synthesis of compound 2-8

To a solution of compound 2-7 (3.10 g, 5.65 mmol, 1.00 eq) in EtOH (30 mL) was added Pd/C (0.6 g, 1.13 mmol, purity: 10%, 0.20 eq) under N₂ atmosphere, and the mixture was degassed under vacuum and replaced three times with H₂. The mixture was stirred under H₂ atmosphere (20 psi) at 25 °C for 34 h. After LCMS showed that compound 2-7 had been consumed and a major peak had been formed, the reaction system was filtered through celite. The filter cake was washed with 10 mL of ethanol, and the filtrate was concentrated under reduced pressure to remove the solvent. To the residue was added THF (30 mL), and the mixture was distilled to remove the solvent. This process was repeated three times. The residue was purified by silica gel column chromatography (dichloromethane/methanol = 92/8) to give compound 2-8 (1.80 g, 2.83 mmol, yield: 48.8%, purity: 81.4%) as a yellow oil. MS m/z (ESI): 519[M+H]⁺; ¹H NMR (400 MHz CDCl₃), δ ppm 8.04 (d, *J* = 2.13 Hz, 1 H), 7.47 (dd, *J* = 8.44, 2.31 Hz, 1 H), 7.20 - 7.27 (m, 1 H), 6.80 - 6.86 (m, 2 H), 6.52 (d, *J* = 8.51 Hz, 1 H), 4.98 (s, 2 H), 4.70 (s, 2 H), 4.16 - 4.26 (m, 4 H), 3.54 - 3.70 (m, 2 H), 2.12 (s, 6 H), 1.16 - 1.32 (m, 6 H).

### Step 8: synthesis of compound 2-9

To a solution of compound 2-8 (1.20 g, 1.16 mmol, 1.00 eq) in THF (2 mL) was added C₂H₅NCO (822 mg, 11.6 mmol, 916 µL, 10.0 eq), and the mixture was stirred at 50 °C for 12 h. After LCMS showed that compound 2-8 had been consumed and a major peak had been formed, the reaction system was concentrated under reduced pressure to remove the solvent to give a residue. The residue was purified by silica gel column chromatography (dichloromethane/methanol = 10/1) to give compound 2-9 (1.01 g, crude) as a yellow oil. MS m/z (ESI): 590[M+H]⁺; ¹H NMR (400 MHz CDCl₃), δ ppm 9.17 (s, 1 H), 8.80 (s, 1 H), 8.19 (d, *J* = 2.00 Hz, 1 H), 7.66 (dd, *J* = 8.50, 2.25 Hz, 1 H), 7.22 - 7.29 (m, 2 H), 6.93 (d, *J* = 8.38 Hz, 1 H), 6.82 - 6.86 (m, 2 H), 5.00 (s, 2 H), 4.18 - 4.29 (m, 4 H), 3.39 - 3.46 (m, 2 H), 3.15 - 3.34 (m, 2 H), 2.17 (s, 6 H), 1.32 (t, *J* = 7.13 Hz, 3 H), 1.23 - 1.27 (m, 3 H), 1.15 - 1.19 (m, 3 H).

### Step 9: synthesis of compound 2-10

Compound 2-9 (1.01 g, 1.72 mmol, 1.00 eq) in EtOH (10 mL) was added to a NaOH (2 M, 4.29 mL, 5.00 eq) solution, and the mixture was stirred at 60 °C for 2 h. After LCMS showed that compound 2-9 had been consumed and a major peak had been formed, the reaction solution was adjusted to pH 5-7 with HCl (1 mol/L, about 8 mL) at 0 °C. The mixture was concentrated under reduced pressure and lyophilized to remove the solvent to give a residue. The residue was purified by silica gel column chromatography (dichloromethane/methanol = 87/13) to give compound 2-10 (798 mg, 1.20 mmol, yield: 70.1%, purity: 84.7%) as a yellow solid. MS m/z (ESI): 562[M+H]⁺; ¹H NMR (400 MHz CDCl₃), δ ppm 9.43 (s, 1 H), 7.97 - 8.04 (m, 2 H), 7.51 - 7.60 (m, 2 H), 7.36 - 7.43 (m, 1 H), 7.04 (t, *J* = 8.00 Hz, 2 H), 4.89 (s, 2 H), 4.01 - 4.05 (m, 2 H), 3.11 - 3.21 (m, 3 H), 2.94 - 3.01 (m, 1 H), 2.44 (s, 6 H), 1.06 - 1.10 (m, 3 H), 0.94 - 1.04 (m, 3 H).

### Step 10: synthesis of compound 2-11

Compound 2-10 (220 mg, 356 µmol, 1.00 eq) and 3-amino-6-methoxypyridazine (80.2 mg, 641 µmol, 1.80 eq) were dissolved in DMF (6 mL), DIEA (460 mg, 3.56 mmol, 620 µL, 10.00 eq) and T₃P (680 mg, 1.07 mmol, 635 µL, purity: 50%, 3.00 eq) were added, and the mixture was mixed with stirring at 25 °C for 17 h. Compound 12a (89.1 mg, 712 µmol, 2.00 eq), DIEA (460 mg, 3.56 mmol, 620 µL, 10.00 eq), and T₃P (680 mg, 1.07 mmol, 635 µL, purity: 50%, 3.00 eq) were added, and the mixture was stirred at 25 °C for 1 h. Compound 12a (89.1 mg, 712 µmol, 2.00 eq), DIEA (460 mg, 3.56 mmol, 620 µL, 10 eq), and T₃P (680 mg, 1.07 mmol, 635 µL, purity: 50%, 3.00 eq) were additionally added, and the mixture was stirred at 25 °C for another 1 h. After LCMS showed that compound 2-10 had been consumed and a major peak had been formed, the mixture was poured into water (12 mL). The resulting mixture was extracted with ethyl acetate (3 × 10.0 mL). The organic phase was washed with brine (3 × 30.0 mL), dried over anhydrous sodium sulfate, filtered, and concentrated to give a residue to give compound 2-11 (270 mg, crude) as a yellow oil. MS m/z (ESI): 669[M+H]⁺.

### Step 11: synthesis of compound 2

MeONa (218 mg, 4.04 mmol, 10.00 eq) was added to a solution of compound 2-11 (270 mg, 404 µmol, 1.00 eq) in MeOH (5 mL) at 0 °C, and the mixture was stirred at 25 °C for 14 h. After LCMS showed that compound 2-11 had been consumed and many peaks had been formed, the reaction solution was quenchced with an aq. NH₄Cl solution (13 mL). Then the mixture was extracted with ethyl acetate (3 × 10.0 mL) and chloromethane (3 × 10.0 mL). The organic phase was washed with brine (50.0 mL), dried over anhydrous sodium sulfate, filtered, and concentrated to give a residue. The residue was purified by silica gel column chromatography (dichloromethane/methanol = 10/1) and column chromatography (Welch xtimate C18 150 × 25 mm × 5 µm, mobile phase: [water (NH₄HCO₃)-ACN]; B%: 10%-85%, 12 min) to give compound 2 (25.0 mg, 40.06 µmol, yield: 9.92%, purity: 99.8%) as a white solid. MS m/z (ESI): 562[M+H]⁺; ¹H NMR (400 MHz CD₃OD), δ ppm 8.31 (d, *J* = 2.13 Hz, 1 H), 7.82 (dd, *J* = 8.63, 2.38 Hz, 1 H), 7.64 (d, *J* = 9.13 Hz, 1 H), 7.32 - 7.49 (m, 2 H), 7.19 (d, *J* = 8.63 Hz, 1 H), 6.93 - 7.07 (m, 2 H), 5.26 - 5.49 (m, 2 H), 4.14 (s, 3 H), 3.29 - 3.36 (m, 4 H), 2.07 (s, 6 H), 1.19 (t, *J* = 7.25 Hz, 3 H). ¹⁹F NMR: (400 MHz CDCl₃) δ - 114.827 ppm.

### Example 3. Synthesis of Compound 3

### Synthesis of fragment 1:

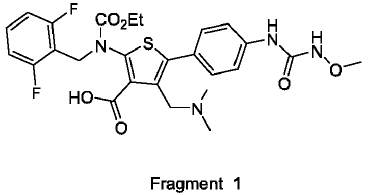

### Synthetic route:

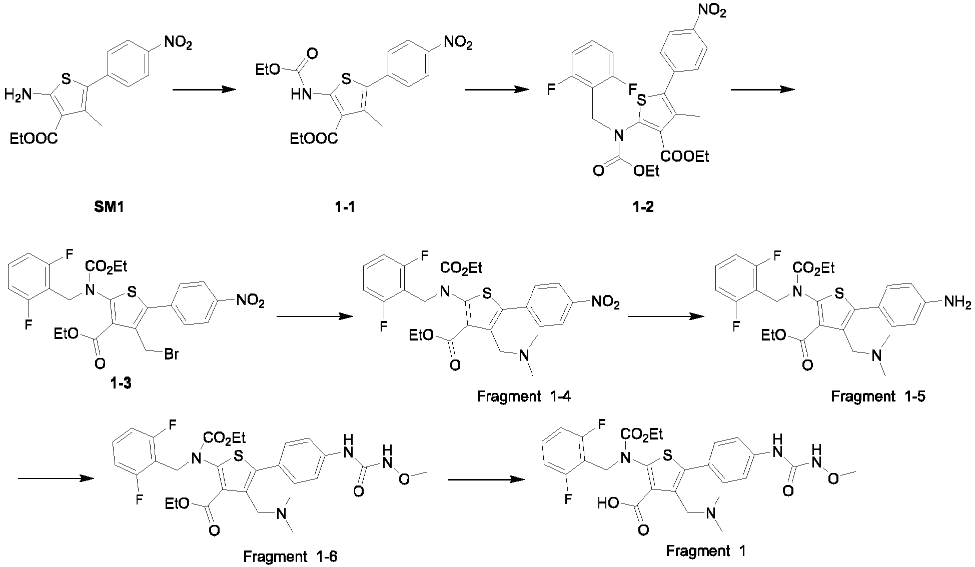

### Steps 1-3: compounds 1-1, 1-2, and 1-3 were synthesized by the same method as that described in Example 1.

### Step 4: synthesis of fragment 1-4

Compound 1-3 (56.5 g, 96.85 mmol, 1 eq), dimethylamine (23.69 g, 290.54 mmol, 26.62 mL, 3 eq, HCl), and DMF (245 mL) were added to a 500 mL single-necked round-bottomed flask at 25 °C. Then triethylamine (39.20 g, 387.39 mmol, 53.92 mL, 4 eq) was added, and the mixture was stirred for reaction for 2 h. The reaction was quenched by adding water (500 mL), extracted with ethyl acetate (200 mL × 3), and subjected to liquid separation. The organic phase was collected, washed with water (300 mL), dried over anhydrous Na₂SO₄, and purified by column chromatography (SiO₂, petroleum ether/ethyl acetate = 10/1 to 0/1) to give fragment 1-4 (38.97 g, 71.03 mmol, yield: 73.34%) as a pale yellow solid. MS m/z (ESI): 548[M+H]⁺; ¹H NMR (400 MHz CDCl₃), δ ppm 1.11 - 1.36 (m, 6H), 2.08 (s, 6H), 3.53 (s, 2H), 4.23 (m, 4H), 5.03 (s, 2H), 6.82 - 6.90 (t, *J* = 7.60 Hz, 2H), 7.23 - 7.31 (m, 1H), 7.67 (d, *J* = 8.80 Hz, 2H), 8.25 (d, *J* = 8.80 Hz, 2H).

### Step 5: synthesis of fragment 1-5

To a solution of fragment 1-4 (30 g, 54.8 mmol) and 4 mol/L hydrochloric acid/dioxane (27.5 mL, 110 mmol) in EtOH (822 mL) was added 10% Pd/C (50% wet, 9.73 g), and the mixture was hydrogenated under H₂ atmosphere (15psi) at 25 °C for 1 h. The mixture was filtered through celite, and the filtrate was neutralized with a saturated aqueous NaHCO₃ solution (200 mL). The combined filtrates were concentrated under vacuum, diluted with ethyl acetate (300 mL) and water (300 mL), and extracted with ethyl acetate (100 mL × 2). The extract was washed with brine, dried (Na₂SO₄), and concentrated under vacuum to give fragment 1-5 (28.79 g, 55.62 mmol, crude) as a pale yellow oil. MS m/z (ESI): 518[M+H]⁺; ¹H NMR (400 MHz CDCl₃), δ ppm 1.17 (s, 3H), 1.31 (t, *J* = 7.20 Hz, 3H), 2.02 (s, 6H), 3.50 (s, 2H), 3.80 (br s, 2H), 4.15 - 4.25 (m, 4H), 5.00 (s, 2 H), 6.66 (d, *J* = 8.38 Hz, 2 H), 6.78 - 6.87 (m, 2H), 7.16 (d, *J* = 8.40 Hz, 2H) 7.20 - 7.26 (m, 1H).

### Step 6: synthesis of fragment 1-6

To a solution of fragment 1-5 (28.79 g, 55.62 mmol, 1 eq) in CH₂Cl₂ (720 mL) were added *N*,*N-*diisopropylethylamine (14.52 g, 112.36 mmol, 19.57 mL, 2.02 eq) and CDI (18.04 g, 111.25 mmol, 2 eq). The mixture was stirred at 25 °C for 12 h, and then cooled to 0 °C. To the mixture were added *N*-(2-methoxyethyl)methylamine (46.36 g, 555.12 mmol, 9.98 eq, HCl) and DIPEA (74.05 g, 572.92 mmol, 99.79 mL, 10.3 eq), and the mixture was stirred at 25 °C for 12 h. The reaction mixture was washed with a saturated aqueous NaHCO₃ solution (200 mL). The organic layer was separated, and the aqueous layer was extracted with CH₂Cl₂ (200 mL × 2). The combined organic phase was washed with brine (400 mL), dried over Na₂SO₄, and concentrated under vacuum. The residue was purified by column chromatography (SiO₂, dichloromethane:methanol = 30:1-5:1) to give fragment 1-6 (24.21 g, 40.99 mmol, yield: 69.34%) as a pale yellow oil. MS m/z (ESI): 591[M+H]⁺; ¹H NMR (400 MHz CDCl₃), δ ppm 1.16 (s, 3H), 1.31 - 1.37 (m, 3H), 2.00 (s, 6 H), 3.50 (s, 1 H), 3.79 (s, 3 H), 4.19 (m, 4H), 4.99 (s, 2H), 6.83 (t, *J* = 7.60 Hz, 2 H), 7.19 - 7.26 (m, 1H), 7.32 (d, *J* = 8.40 Hz, 2H), 7.51 (d, *J* = 8.80 Hz, 2H), 7.71 - 7.92 (m, 2 H).

### Step 7: synthesis of fragment 1

To a solution of fragment 1-6 (24.21 g, 40.99 mmol) in EtOH (500 mL) was added 2 mol/L NaOH (102 mL, 204.95 mmol). After stirring at 60 °C for 6 h, 1 mol/L HCl (205 mL, 205 mmol) was added at 0 °C, and the mixture was concentrated under vacuum. The residue was dissolved in 200 mL of ethanol and 200 mL of toluene, and the resulting solution was concentrated. The residue was diluted with absolute ethanol (200 mL) and filtered, and the filtrate was concentrated under reduced pressure. To the residue was added methyl t-butyl ether (100 mL). The precipitate was collected by filtration, washed with methyl t-butyl ether (50 mL), and dried to give fragment 1 (17.59 g, 31.3 mmol, 76.3%) as a pale yellow solid. MS m/z (ESI): 563[M+H]⁺; ¹H NMR (400 MHz CDCl₃), δ ppm 1.05 - 1.10 (m, 3H), 2.44 (s, 6H), 3.62 (s, 3H), 3.90 - 4.17 (m, 4H), 4.90 (s, 2 H), 7.04 (t, *J* = 8.00 Hz, 2H), 7.16 (d, *J* = 8.00 Hz, 2H), 7.34 - 7.44 (m, 2H), 7.64 - 7.73 (d, *J* = 8.00 Hz, 2H), 9.22 (br s, 1H), 9.71 (br s, 1H).

### Synthesis of compound 3:

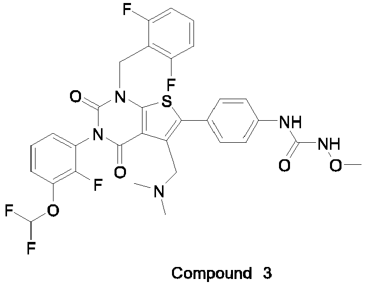

### Synthetic route:

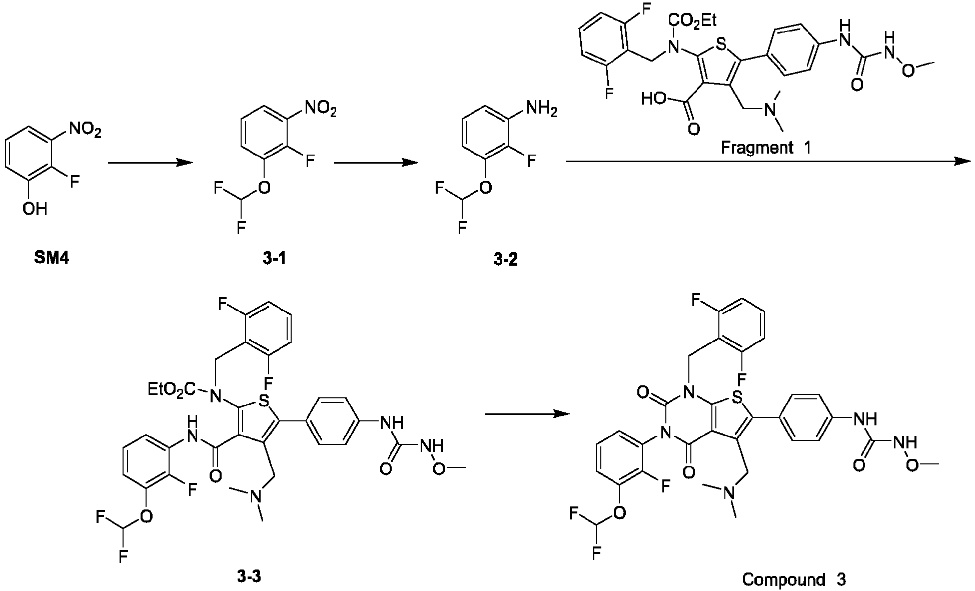

### Synthesis of compound 3-1:

To a solution of sodium difluorochloroacetate (17.4 g, 114.5 mmol) in DMF (25.0 mL) and water (6.00 mL) was added K₂CO₃ (8.80 g, 63.6 mmol), then a solution of SM4 (5.00 g, 31.8 mmol) in DMF (25.0 mL) was added dropwise, and the system was stirred at 110 °C for 12 h. After LC-MS showed that SM4 had been completely consumed, the reaction mixture was poured into water (100 mL). The resulting mixture was extracted with ethyl acetate (200 mL × 3). The combined organic layer was concentrated under reduced pressure to give a residue. The residue was purified by column chromatography (SiO₂, petroleum ether/ethyl acetate = 10/1) to give compound 3-1 (0.30 g, 1.45 mmol, yield: 4.55%) as a yellow oil. ¹H NMR (400 MHz CDCl₃), δ ppm 7.96 - 7.92 (m, 1H), 7.58 - 7.56 (t, *J* = 7.2 Hz 1H), 7.32 - 4.29 (m, 1H), 3.53 (t, *J* = 144 Hz, 1H).

### Synthesis of compound 3-2:

To a solution of compound 3-1 (0.30 g, 1.45 mmol) in MeOH (5.00 mL) was added Pd/C (0.03 g, purity: 10%), and the mixture was charged with hydrogen and stirred at 25 °C for 16 h. After LC-MS showed that compound 3-1 had been completely consumed, the mixture was filtered and concentrated under reduced pressure to give a crude product of compound 3-2 (172 mg, 971 µmol, yield: 67.0%) as a yellow solid. MS m/z (ESI): 178[M+H]⁺; ¹H NMR (400 MHz CDCl₃), δ ppm 6.89 - 6.86 (m, 1H), 6.66 - 6.53 (m, 1H), 6.71 - 6.34 (t, *J* = 147.6 Hz, 1H).

### Step 1: synthesis of compound 3-3

To a solution of compound 3-2 (170 mg, 959 µmol) and fragment 1 (300 mg, 533.25 µmol) in ACN (5.00 mL) were added T₃P (1.02 g, 1.60 mmol, 951.43 µL, purity: 50%) and Et₃N (134 mg, 1.33 mmol, 185 µL), and the mixture was stirred at 40 °C for 2 h. After LC-MS showed that fragment 1 had been completely consumed, the reaction mixture was adjusted to pH 5-6 with a sodium carbonate solution at 0 °C, and then diluted with 20.0 mL of water and extracted with ethyl acetate (30 mL × 3). The organic phase was dried over anhydrous sodium sulfate, filtered, and concentrated under reduced pressure. The residue was purified by column chromatography (SiO₂, dichloromethane:methanol = 20/1-10/1) to give compound 3-3 (130 mg, 145 µmol, yield: 27.3%, purity: 81.0%) as a yellow oil. MS m/z (ESI): 722[M+H]⁺.

### Step 2: synthesis of compound 3

To a solution of compound 3-3 (130 mg, 180 µmol) in methanol (2.00 mL) was added sodium methoxide (97.3 mg, 1.80 mmol), and the mixture was stirred at 25 °C for 2 h. After LC-MS showed that compound 3-3 had been completely consumed, the reaction system was subjected to extraction and liquid separation in 10.0 mL of an aqueous ammonium chloride solution and 20.0 mL of an ethyl acetate solution. The organic phase was concentrated under reduced pressure to give a residue. The residue was purified by column chromatography (SiO₂, dichloromethane:MeOH = 1/30-1/10) to give compound 3 (71.0 mg, 99.8 µmol, yield: 55.4%) as a yellow solid. MS m/z (ESI): 676[M+H]⁺; ¹H NMR (400 MHz CD₃OD), δ ppm 7.71 - 7.69 (d, *J* = 8.80 Hz, 2H), 7.46 - 7.33 (m, 6H), 7.04 - 7.02 (t, *J* = 8.40 Hz, 2H), 7.06 - 6.73 (t, *J* = 131.6 Hz, 1H), 5.55 - 5.51 (d, *J* = 16.0 Hz, 1H), 5.41 - 5.37 (d, *J* = 16.0 Hz, 1H), 4.61(s, 1H), 4.26 - 4.17 (m, 2H), 3.74 (s, 3H), 2.17 (s, 6H).

### Example 4. Synthesis of Fragment 2

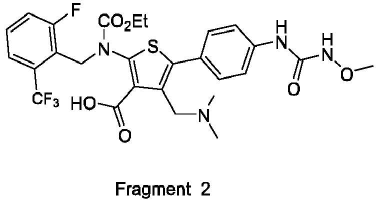

### Synthetic route:

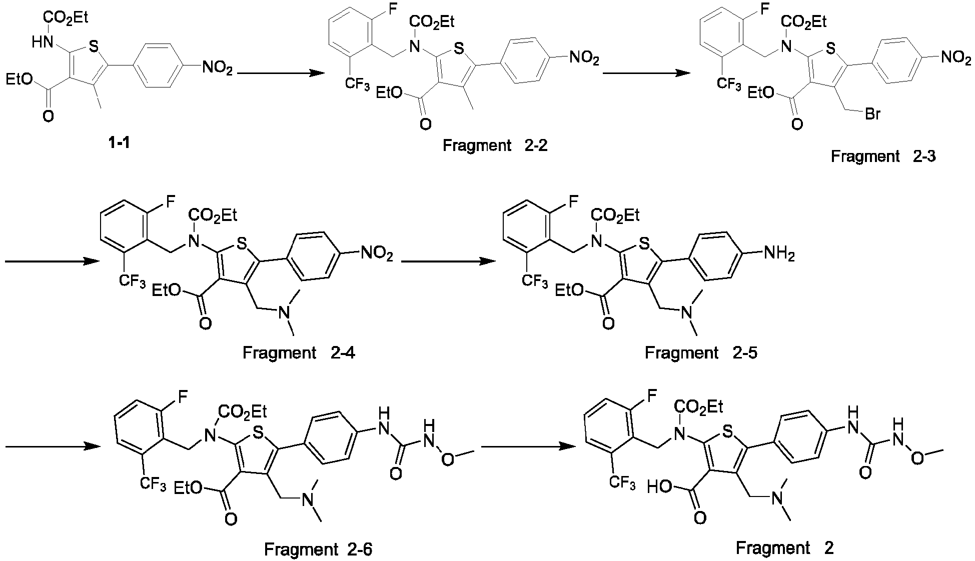

### Step 1: synthesis of fragment 2-2

To a solution of compound 1-1 (5 g, 13.21 mmol, 1 eq) in DMF (150 mL) were added K₂CO₃ (2.01 g, 14.53 mmol, 1.1 eq) and KI (2.41 g, 14.53 mmol, 1.1 eq). Then, to the mixture was added a solution of 2-fluoro-6-(trifluoromethyl)bromobenzyl (55.46 g, 341.14 mmol, 1.2 eq) in DMF (190 mL). The mixture was stirred at 25 °C for 17 h. The precipitate thus produced was filtered off, and the filtrate was concentrated under vacuum. The residue was diluted with ethyl acetate (700 mL) and water (700 mL), and extracted with ethyl acetate (300 mL × 2). The extract was washed with 500 mL of water and 500 mL of brine, dried over anhydrous Na₂SO₄, and concentrated under reduced pressure. The residue was purified by column chromatography (SiO₂, petroleum ether/ethyl acetate = 10-1-2/1) and TLC (petroleum ether: dichloromethane = 2:1, plate 2, Rf = 0.67) to give fragment 2-2 (7.23 g, 10.30 mmol, yield: 77.95%, purity: 79%) as a yellow oil. MS m/z (ESI): 555[M+H]⁺.

### Step 2: synthesis of fragment 2-3

Fragment 2-2 (7.21 g, 13.00 mmol, 1 eq), AIBN (213.51 mg, 1.30 mmol, 0.1 eq), and NBS (2.89 g, 16.25 mmol, 1.25 eq) were reacted in ethyl acetate (100 mL) at 80 °C for 2 h. NBS (1.39 g, 7.80 mmol, 0.6 eq) and AIBN (106.76 mg, 650.12 µmol, 0.05 eq) were additionally added, and then the mixture was reacted at 80 °C for 2 h. NBS (1.39 g, 7.80 mmol, 0.6 eq) and AIBN (106.76 mg, 650.12 µmol, 0.05 eq) were additionally added, and then the mixture was reacted at 80 °C for 2 h. After cooling to room temperature, the reaction mixture was diluted with ethyl acetate (200 mL) and water (300 mL), and extracted with ethyl acetate (200 mL × 2). The extract was washed with 300 mL of water and 500 mL of brine, dried (Na₂SO₄), and concentrated under reduced pressure to give a crude product of fragment 2-3 (7.22 g, crude), which was directly used in the next step. MS m/z (ESI): 633 [M+H]⁺.

### Step 3: synthesis of fragment 2-4

Fragment 2-3 (7.20 g, 11.37 mmol, 1 eq), dimethylamine (3.84 g, 34.11 mmol, 4.32 mL, purity: 40%, 3 eq), and DMF (300 mL) were added to a 500 mL single-necked round-bottomed flask at 25 °C. Triethylamine (4.60 g, 45.48 mmol, 6.33 mL, 4 eq) was added, and the mixture was stirred for reaction for 2 h. The reaction was quenched by adding water (700 mL), extracted with ethyl acetate (200 mL × 3), and subjected to liquid separation. The organic phase was collected, washed with water (300 mL), dried over anhydrous Na₂SO₄, and purified by column chromatography (SiO₂, petroleum ether/ethyl acetate = 10/1 to 0/1) to give fragment 2-4 (5.12 g, 7.37 mmol, yield: 64.81%, purity: 86%) as a pale yellow solid. MS m/z (ESI): 598[M+H]⁺.

### Step 4: synthesis of fragment 2-5

To a solution of fragment 2-4 (5.12 g) and 4 mol/L hydrochloric acid/1,4-dioxane (12.5 mL, 110 mmol) in EtOH (200 mL) was added 10% Pd/C (50% wet, 5.0 g), and the mixture was reacted at 25 °C for 6 h under H₂ atmosphere (15 psi). The reaction system was filtered through celite, and the filtrate was neutralized with a saturated aqueous NaHCO₃ solution (200 mL). The filtrate was concentrated under vacuum, diluted with ethyl acetate (300 mL) and water (300 mL), and extracted with ethyl acetate (100 mL × 2). The organic phase was washed with brine, dried (Na₂SO₄), and concentrated under vacuum to give fragment 2-5 (28.79 g, 55.62 mmol, yield: 102%) as a pale yellow oil. MS m/z (ESI): 568[M+H]⁺.

### Step 5: synthesis of fragment 2-6

To a solution of fragment 2-5 (4.18 g, 7.36 mmol, 1 eq) in CH₂Cl₂ (100 mL) were added *N*,*N-*diisopropylethylamine (14.52 g, 112.36 mmol, 19.57 mL, 2.02 eq) and CDI (4.78 g, 29.46 mmol, 4 eq). The mixture was stirred at 25 °C for 12 h, and then cooled to 0 °C. The mixture was stirred at 25 °C for 12 h, and then cooled to 0 °C. To the mixture was added methoxyamine hydrochloride (6.14 g, 73.50 mmol, 5.58 mL, 9.98 eq) and DIPEA (9.80 g, 75.85 mmol, 13.21 mL, 10.3 eq). The resulting mixture was stirred at 25 °C for 12 h. The reaction solution was washed with a saturated aqueous NaHCO₃ solution (200 mL). The organic layer was separated, and the aqueous layer was extracted with CH₂Cl₂ (200 mL × 2). The combined organic phase was washed with brine (400 mL), dried over anhydrous Na₂SO₄, and concentrated under vacuum. The residue was purified by column chromatography (SiO₂, dichloromethane:methanol = 30:1-5:1) to give fragment 2-6 (4.22 g, 6.59 mmol, yield: 89.45%) as a pale yellow oil. MS m/z (ESI): 641[M+H]⁺.

### Step 6: synthesis of fragment 2

2 mol/L NaOH (102 mL, 204.95 mmol) was added to a solution of fragment 2-6 (4.22 g, 6.59 mmol, 1 eq) in EtOH (500 mL). After stirring at 40 °C for 12 h, 1 mol/L HCl (205 mL, 205 mmol) was added at 0 °C, and the mixture was concentrated under vacuum. The residue was dissolved in 200 mL of ethanol and 200 mL of toluene, and the resulting solution was concentrated. The residue was diluted with absolute ethanol (200 mL) and filtered, and the filtrate was concentrated under reduced pressure. To the residue was added methyl *t*-butyl ether (100 mL). The precipitate was collected by filtration, washed with methyl *t*-butyl ether (50 mL), and dried to give fragment 2 (2.0 g, 2.87 mmol, yield: 43.62%, purity: 88%) as a pale yellow solid. MS m/z (ESI): 613[M+H]⁺.

### Example 5. Synthesis of Compound 3A

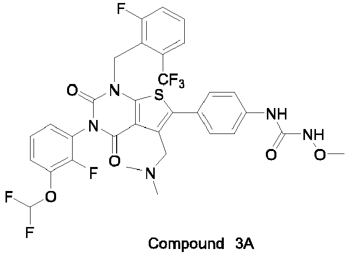

### Synthetic route:

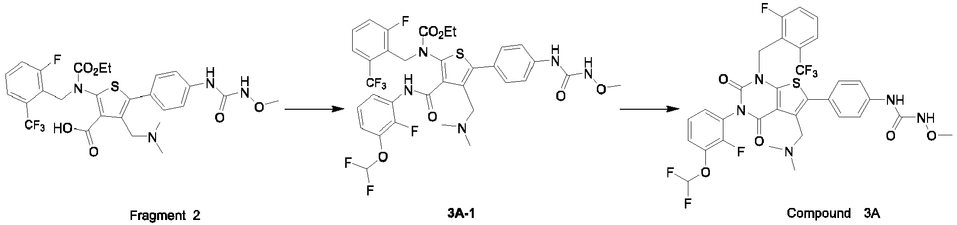

### Step 1: synthesis of compound 3A-1

To a solution of fragment 2 (700 mg, 1.14 mmol, 1 eq) and 3-difluoromethoxy-2-fluoroaniline (364.31 mg, 2.06 mmol, 1.8 eq) in ACN (50 mL) were added T₃P (1.82 g, 2.86 mmol, 1.70 mL, purity: 50%, 2.5 eq) and Et₃N (346.88 mg, 3.43 mmol, 477.14 µL, 3 eq), and the mixture was mixed with stirring at 40 °C for 2 h. Then the reaction mixture was adjusted to pH 5-6 with a sodium carbonate solution at 0 °C, diluted with 100.0 mL of water, extracted with ethyl acetate (100 mL × 3), and concentrated to give compound 3A-1 (300 mg, crude), which was directly used in the next step without purification. MS m/z (ESI): 772[M+H]⁺.

### Step 2: synthesis of compound 3A

To a solution of compound 3A-1 (300 mg, 388 µmol) in methanol (5.00 mL) was added sodium methoxide (209 mg, 3.88 mmol), and the mixture was stirred at 25 °C for 2 h. After LC-MS showed that compound 3A-1 had been completely consumed, the reaction mixture was subjected to extraction and liquid separation in 10.0 mL of an aqueous ammonium chloride solution and 20.0 mL of an ethyl acetate solution. The organic phase was concentrated under reduced pressure to give a residue. The residue was purified by reversed-phase high-performance liquid chromatography (column: Xtimate C18 150 × 40 mm × 10 µm; mobile phase: [ water (NH₃H₂O + NH₄HCO₃)-ACN]; B%: 50%-80%, 10 min). The crude product was purified by reversed-phase high-performance liquid chromatography (column: Welch Xtimate C18 150 mm × 30 mm × 5 µm, mobile phase: [water (TFA)-ACN], B%: 15%-55%, 10 min) to give compound 3A (191 mg, 257.95 µmol, yield: 28.44%, purity: 98%) as a yellow solid. MS m/z (ESI): 726[M+H]⁺; ¹H NMR (400 MHz CD₃OD), δ ppm 7.66 - 7.76 (m, 3 H) 7.57 - 7.65 (m, 1 H) 7.43 - 7.52 (m, 2 H) 7.09 - 7.13 (m, 1 H) 6.89 - 6.95 (m, 1 H) 6.69 - 6.76 (m, 1 H) 5.74 (d, *J* = 16.00 Hz, 1 H) 5.54 (d, *J* = 16.00 Hz, 1 H) 4.39 - 4.54 (m, 2 H) 3.75 (s, 3 H) 2.70 - 2.80 (m, 6 H).

### Example 6. Synthesis of Compound 4

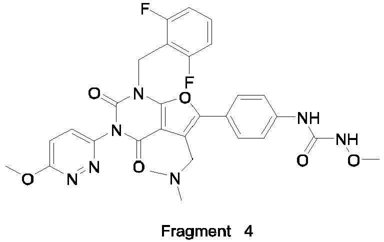

### Synthetic route:

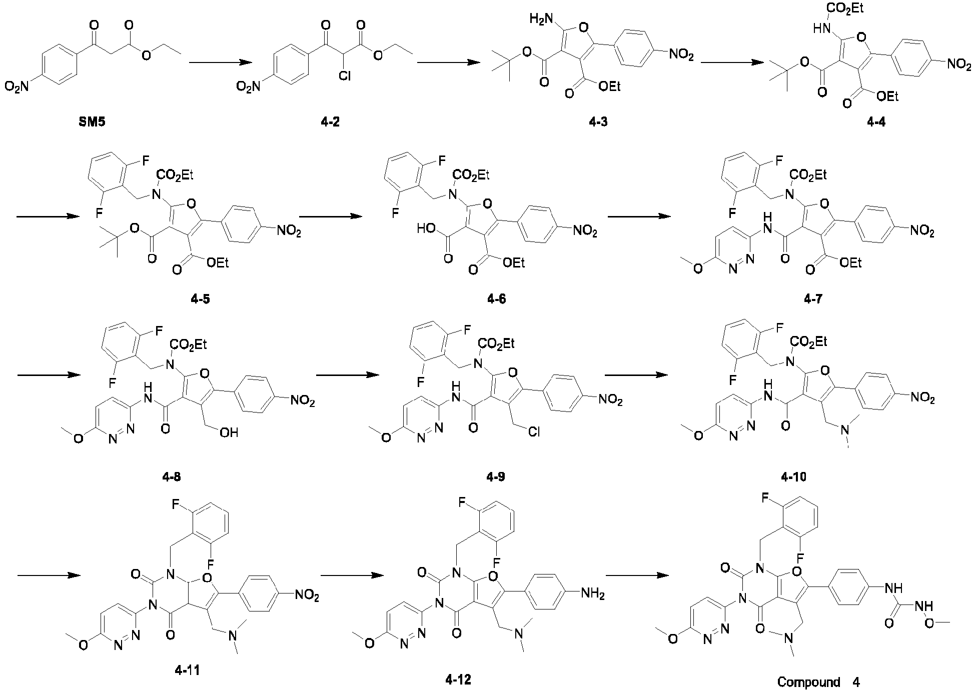

### Step 1: synthesis of compound 4-2

To a suspension of SM5 (21.0 g, 88.5 mmol) in anhydrous toluene (100 mL) was added sulfuryl chloride (15.5 g, 115 mmol, 11.5 mL), and the mixture was stirred at 85 °C for 12 h, and then stirred at 100 °C for 4 h. After LCMS and TLC (petroleum ether:EtOAc = 5:1, Rf = 0.31) showed that the starting material had been almost completely consumed, the mixture was cooled to 25 °C and quenched with ice water (50.0 mL). The organic layer was separated, and the aqueous layer was extracted with toluene (100 mL). The combined organic layer was dried over anhydrous Na₂SO₄ and concentrated under reduced pressure to remove the solvent to give compound 4-2 (23.0 g, 69.4 mmol, yield: 78.4%, purity: 82%) as a yellow viscous oil, which was directly used in the next step without further purification. ¹H NMR (400 MHz CDCl₃), δppm 8.35 (d, *J* = 8.8 Hz, 2H), 8.17 (d, *J* = 8.8 Hz, 2H), 5.56 (s, 1H), 4.34 - 4.28 (m, 2H), 1.26 (t, *J* = 7.2 Hz, 3H).

### Step 2: synthesis of compound 4-3

Solution 1: NaH (3.39 g, 84.6 mmol, purity: 60%) was added to a solution of t-butyl 2-cyanoacetate (11.9 g, 84.6 mmol, 12.1 mL) in THF (120 mL) at 0 °C, and the mixture was stirred at 0-25 °C for 30 min. Fresh solution 1 was added dropwise to a solution of compound 4-2 (23.0 g, 84.6 mmol) in THF (120 mL) over 30 min. The mixture was stirred at 25 °C for 16 h, and then stirred at 70 °C for 24 h. After LCMS showed that the starting material had been completely consumed, the mixture was concentrated under reduced pressure to remove the solvent. The mixture was purified by silica gel column chromatography to give compound 4-3 (18.5 g, 42.7 mmol, yield: 50.5%, purity: 87.0%) as an orange solid. MS m/z (ESI): 377[M+H]⁺; ¹H NMR (400 MHz CDCl₃), δ8.20 (d, *J* = 9.2 Hz, 2H), 7.63 (d, *J* = 9.2 Hz, 2H), 5.76 (br.s, 2H), 4.43 - 4.38 (m, 2H), 1.53 (s, 9H), 1.39 (t, *J* = 7.2 Hz, 3H).

### Step 3: synthesis of compound 4-4

To a solution of compound 4-3 (5.00 g, 13.3 mmol) and Et₃N (4.03 g, 39.8 mmol, 5.55 mL) in THF (100 mL) was added ClCO₂Et (1.85 g, 17.0 mmol, 1.62 mL), and the mixture was stirred at 25 °C for 16 h. ClCO₂Et (1.69 g, 15.5 mmol, 1.48 mL) was additionally added, and the mixture was stirred at 25 °C for 4 h. The reaction mixture was concentrated under reduced pressure to remove the solvent. The residue was dissolved in 50 mL of methanol, K₂CO₃ (2.75 g, 19.9 mmol) was added, and the mixture was stirred at 50 °C for 16 h. After LCMS showed that the starting material had been completely consumed, the mixture was diluted with water (200 mL) and extracted with ethyl acetate (150 mL × 3). The organic layer was washed with water (150 mL) and brine (100 mL), dried over Na₂SO₄, and concentrated under reduced pressure to remove the solvent. The residue was purified by silica gel column chromatography to give compound 4-4 (4.20 g, 8.71 mmol, yield: 65.5%, purity: 93.0%) as a yellow solid. MS m/z (ESI): 449[M+H]⁺; ¹H NMR (400 MHz CDCl₃), δppm 9.02 (br.s, 1H), 8.24 (d, *J* = 9.2 Hz, 2H), 7.79 (d, *J* = 8.8 Hz, 2H), 4.44 - 4.38 (m, 2H), 4.33 - 4.30 (m, 2H), 1.55 (s, 9H), 1.41 - 1.35 (m, 6H).

### Step 4: synthesis of compound 4-5

To a solution of compound 4-4 (4.50 g, 10.0 mmol) in DMF (50.0 mL) were added K₂CO₃ (1.53 g, 11.04 mmol) and KI (1.83 g, 11.0 mmol), then 2,6-difluorobenzyl chloride (1.96 g, 12.0 mmol) was added, and the mixture was stirred at 25 °C for 3 h. After LCMS showed that the starting material had been completely consumed, the mixture was poured into water (100 mL). The resulting mixture was extracted with ethyl acetate (50 mL × 3). The organic layer was washed with water (50 mL × 2), dried over Na₂SO₄, and concentrated under reduced pressure to remove the solvent. The residue was purified by silica gel column chromatography (petroleum ether:EtOAc = 10:1, Rf = 0.46) to give compound 4-5 (4.90 g, 8.14 mmol, yield: 81.1%, purity: 95.5%) as a yellow oil. MS m/z (ESI): 575[M+H]⁺; ¹H NMR (400 MHz CDCl₃), δppm 8.23 (d, *J* = 8.8 Hz, 2H), 7.74 (d, *J* = 8.8 Hz, 2H), 7.27 - 7.24 (m, 1H), 6.87 (t, *J* = 8.0 Hz, 2H), 5.00 (s, 2H), 4.42 - 4.37 (m, 2H), 4.25 - 4.20 (m, 2H), 1.49 (s, 9H), 1.36 (t, *J* = 7.2 Hz, 3H), 1.25 - 1.23 (m, 3H).

### Step 5: synthesis of compound 4-6

To a solution of compound 4-5 (4.90 g, 8.53 mmol) in CH₂Cl₂ (25.0 mL) was added TFA (9.24 g, 81.0 mmol, 6.00 mL), and the mixture was stirred at 25 °C for 3 h. After LCMS showed that the starting material had been completely consumed, the mixture was concentrated under reduced pressure to remove the solvent. The residue was dissolved in 50 mL of CH₂Cl₂, and then the solvent was removed. The above process was repeated 3 times to give compound 4-6 (5.20 g, 8.22 mmol, yield: 96.4%, TFA) as a brown oil, which was directly used in the next step without purification. MS m/z (ESI): 519[M+H]⁺; ¹H NMR (400 MHz CDCl₃), δppm 8.28 (d, *J* = 8.8 Hz, 2H), 7.70 (d, *J* = 8.8 Hz, 2H), 7.31 - 7.25 (m, 1H), 6.87 (t, *J* = 8.0 Hz, 2H), 5.02 (s, 2H), 4.41 - 4.35 (m, 2H), 4.26 - 4.23 (m, 2H), 1.26 (t, *J* = 7.2 Hz, 6H).

### Step 6: synthesis of compound 4-7

To a solution of compound 4-6 (4.00 g, 7.72 mmol) and 3-amino-6-methoxypyridazine (2.00 g, 15.9 mmol) in tetrahydrofuran (80.0 mL) were added *N*-methylimidazole (2.53 g, 30.8 mmol, 2.46 mL) and *N*,*N*,*N*',*N*'-tetramethylchloroformamidinium hexafluorophosphate (4.33 g, 15.4 mmol), and the mixture was mixed with stirring at 25 °C for 16 h. After LCMS showed that the starting material had been completely consumed, the mixture was concentrated under reduced pressure to remove the solvent. The residue was purified by silica gel column chromatography (petroleum ether: ethyl acetate = 2:1-0:1) to give compound 4-7 (2.90 g, 4.27 mmol, yield: 55.3%, purity: 92.0%) as a yellow oil. MS m/z (ESI): 626[M+H]⁺; ¹H NMR (400 MHz CDCl₃), δppm 10.76 (br.s, 1H), 8.49 (d, *J* = 9.6 Hz, 1H), 8.28 (d, *J* = 8.8 Hz, 2H), 7.83 (d, *J* = 8.4 Hz, 2H), 7.25 - 7.23 (m, 1H), 7.09 (d, *J* = 9.2 Hz, 1H), 6.84 (t, *J* = 8.0 Hz, 2H), 5.04 (s, 2H), 4.35 - 4.29 (m, 4H), 4.13 (s, 3H), 1.31 - 1.18 (m, 6H).

### Step 7: synthesis of compound 4-8

To a solution of compound 4-7 (2.50 g, 4.00 mmol) in THF (20 mL) and EtOH (20 mL) was added LiBH₄ (435 mg, 19.9 mmol), and the mixture was stirred at 0 °C for 1 h, and then stirred at 0-25 °C for 3 h. After LCMS showed that the starting material had been completely consumed, the mixture was poured into an aq. NH₄Cl solution (150 mL). The resulting mixture was extracted with ethyl acetate (100 mL × 3). The combined organic layer was washed with brine (50.0 mL), dried over Na₂SO₄, and concentrated under reduced pressure to remove the solvent to give compound 4-8 (2.30 g, 3.19 mmol, yield: 79.9%, purity: 81.0%) as a yellow solid, which was directly used in the next step without purification. MS m/z (ESI): 584[M+H]⁺.

### Step 8: synthesis of compound 4-9

To a solution of compound 4-8 (2.22 g, 3.80 mmol) in CH₂Cl₂ (50.0 mL) at 0 °C was added SOCl₂ (905 mg, 7.61 mmol, 552 µL), and the mixture was stirred at 0-25 °C for 3 h. After LCMS showed that the starting material had been completely consumed, the mixture was concentrated under reduced pressure to remove the solvent to give compound 4-9 (2.30 g, crude) as a yellow solid, which was directly used in the next step without purification. MS m/z (ESI): 602[M+H]⁺.

### Step 9: synthesis of compound 4-10

To a solution of compound 4-9 (2.30 g, 3.82 mmol) in CH₂Cl₂ (50.0 mL) were added Et₃N (3.87 g, 38.2 mmol, 5.32 mL) and dimethylamine hydrochloride (1.56 g, 19.1 mmol), and the mixture was stirred at 25 °C for 16 h. After LCMS showed that the starting material had been completely consumed, the mixture was diluted with CH₂Cl₂ (100 mL), washed with water (50.0 mL × 2), and extracted with CH₂Cl₂ (50.0 mL) to separate the aqueous layer. The combined organic layer was washed with brine (50.0 mL), dried over Na₂SO₄, and concentrated under reduced pressure to remove the solvent. The residue was purified by silica gel column chromatography to give compound 4-10 (1.40 g, 2.13 mmol, yield: 55.8%, purity: 93.0%) as a yellow solid. MS m/z (ESI): 611[M+H]⁺.

### Step 10: synthesis of compound 4-11

To a solution of compound 4-10 (210 mg, 344 µmol) in absolute methanol (20 mL) was added CH₃ONa (186 mg, 3.44 mmol), and the mixture was stirred at 25 °C for 16 h. After LCMS showed that the starting material had been completely consumed and the expected product had been observed, four batches of the mixture were mixed. The mixture was poured into an aq. NH₄Cl solution (150 mL), extracted with ethyl acetate (150 mL × 3), washed with water (100 mL) and brine (100 mL), dried over Na₂SO₄, and concentrated under reduced pressure to remove the solvent. The residue was purified by silica gel column chromatography to give compound 4-11 (530 mg, 884 µmol, yield: 64.3%, purity: 94.2%) as a yellow solid. MS m/z (ESI): 565 [M+H]⁺; ¹H NMR (400 MHz CDCl₃), δppm 8.35 (d, *J* = 8.4 Hz, 2H), 8.07 (d, *J* = 9.2 Hz, 2H), 7.42 - 7.35 (m, 2H), 7.17 (d, *J* = 9.2 Hz, 1H), 6.99 - 6.95 (m, 2H), 5.45 (s, 2H), 4.19 (s, 3H), 4.00 - 3.73 (m, 2H), 2.48 (s, 6H).

### Step 11: synthesis of compound 4-12

To a solution of compound 4-11 (105 mg, 172 µmol) in EtOH (5.00 mL) and THF (5.00 mL) was added Pd/C (50.0 mg, 86.0 µmol, purity: 10%) under N₂ atmosphere, and the mixture was degassed under vacuum and purged 3 times with H₂. The mixture was stirred at 25 °C for 16 h under H₂ atmosphere (15 PSI). After LCMS showed that the starting material had been completely consumed, the mixture was filtered through a pad of celite, and the filter cake was washed with 30 mL of ethanol (EtOH). The filtrate was concentrated under reduced pressure to remove the solvent. The residue was purified by silica gel column chromatography (CH₂Cl₂:MeOH = 10:1, Rf = 0.31) to give compound 4-12 (67.0 mg, 100 µmol, yield: 58.3%, purity: 87.0%) as a brown oil. MS m/z (ESI): 535 [M+H]⁺.

### Step 12: synthesis of compound 4

To a solution of compound 4-12 (50.0 mg, 93.5 µmol) in CH₂Cl₂ (5 mL) were added DIPEA (105 mg, 812 µmol, 141 µL) and CDI (60.6 mg, 374 µmol), and the mixture was stirred at 25 °C for 16 h. DIPEA (120 mg, 935 µmol, 163 µL) and CDI (60.0 mg, 370 µmol) were additionally added, and the mixture was stirred at 25 °C for 2 h. CDI (151 mg, 935 µmol) was added, and the mixture was stirred at 25 °C for 2 h. CDI (151 mg, 935 µmol) was added, and the mixture was stirred at 25 °C for 2 h. CDI (151 mg, 935 µmol) was added, and the mixture was stirred at 25 °C for 2 h. Methoxyamine hydrochloride (78.1 mg, 935 µmol) was added, and the mixture was stirred at 25 °C for 2 h. After LCMS showed that the starting material had been completely consumed, the mixture was diluted with ethyl acetate (50 mL) and washed with water (20 mL × 2) and brine (20 mL). The organic layer was dried over Na₂SO₄ and concentrated under reduced pressure to remove the solvent. The residue was purified by silica gel column chromatography to give compound 4 (35.0 mg, 52.4 µmol, yield: 56.0%, purity: 91.0%) as an off white solid. MS m/z (ESI): 608 [M+H]⁺; ¹H NMR (400 MHz CD₃OD), δppm 7.73 - 7.63 (m, 5H), 7.40 - 7.36 (m, 2H), 7.05 - 7.01 (m, 3H), 5.46 (s, 2H), 4.16 (s, 3H), 3.75 - 3.73 (m, 5H), 2.29 (s, 6H).

### Example 7. Synthesis of Compound 5

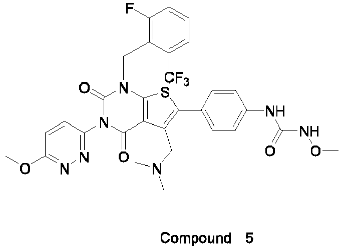

### Synthetic route:

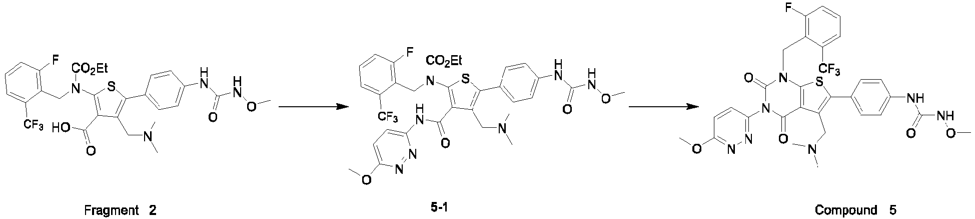

### Step 1: synthesis of compound 5-1

Fragment 2 (700 mg, 1.14 mmol, 1 eq) and 3-amino-6-methoxypyridazine (714.91 mg, 5.71 mmol, 5 eq) were dissolved in a solution of DMF (10 mL), T₃P (5.82 g, 9.14 mmol, 5.44 mL, purity: 50%, 8 eq) and DIEA (2.22 g, 17.14 mmol, 2.99 mL, 15 eq) were added, and the mixture was stirred at 25 °C for 2 h. The reaction mixture was adjusted to pH 5-6 with a sodium carbonate solution at 0 °C, and then diluted with 100.0 mL of water and extracted with ethyl acetate (100 mL × 3). The organic phase was dried over anhydrous sodium sulfate and filtered, and the filtrate was concentrated under reduced pressure. The filtrate was concentrated under reduced pressure to give compound 5-1 (650 mg, crude) as a yellow oil. MS m/z (ESI): 720[M+H]⁺.

### Step 2: synthesis of compound 5

Compound 5-1 (650 mg, 903.15 µmol, 1 eq) was dissolved in MeOH (20 mL), NaOMe (487.91 mg, 9.03 mmol, 10 eq) was added, and the mixture was stirred at 25 °C for 2 h. The reaction mixture was adjusted to pH 5-6 with a sodium carbonate solution at 0 °C, and then diluted with 100.0 mL of water and extracted with ethyl acetate (100 mL × 3). The organic phase was dried over anhydrous sodium sulfate and filtered, and the filtrate was concentrated under reduced pressure. The crude product was purified by preparative high-performance liquid chromatography (column: Xtimate C18 150 × 40 mm × 10 µm; mobile phase: [water (NH₃H₂O + NH₄HCO₃)-ACN]; B%: 40%-70%, 10 min) to give compound 5 (137 mg, 199.31 µmol, yield: 22.07%, purity: 98%) as a yellow solid. MS m/z (ESI): 674[M+H]⁺; ¹H NMR (400 MHz CD₃OD), δppm 7.55 - 7.72 (m, 5 H) 7.34 - 7.48 (m, 4 H) 4.17 (s, 3 H) 3.74 (s, 1 H) 3.64 - 3.87 (m, 1 H) 3.60 - 4.00 (m, 1 H) 2.08 - 2.24 (m, 6 H).

### Example 8. Synthesis of Compound 6

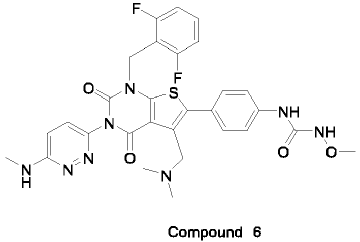

### Synthetic route:

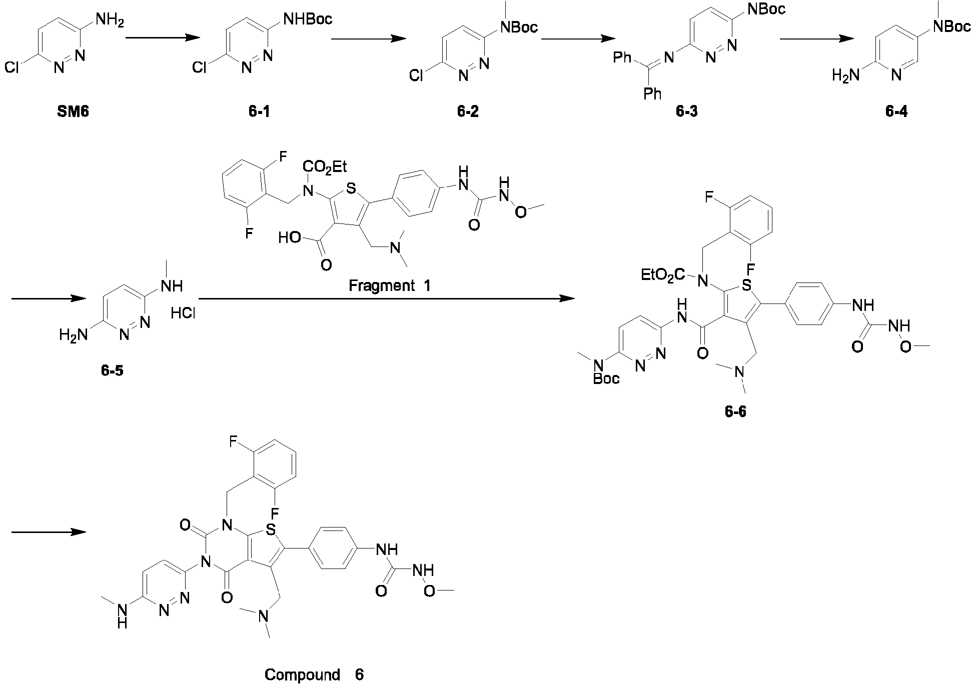

### Step 1: synthesis of compound 6-1

To a solution of SM6 (5.00 g, 38.6 mmol) in dioxane (100 mL) were added DMAP (94.3 mg, 771 µmol) and di-*tert*-butyl dicarbonate (12.6 g, 57.8 mmol, 13.3 mL), and the mixture was stirred at 80 °C for 2 h. After LC-MS showed that the starting material b1 was completely consumed, the reaction mixture was extracted with ethyl acetate to give singly protected and doubly protected amines, which were dissolved in methanol (100 mL), and potassium carbonate (1.26 g, 9.10 mmol) was added. The mixture was stirred at 25 °C until no doubly protected amine was observed by LC-MS. The reaction mixture was poured into water (100.0 mL). The resulting mixture was filtered to give compound 6-1 (4.60 g, 18.2 mmol, yield: 52.0%, purity: 91%) as a yellow solid. MS m/z (ESI): 230[M+H]⁺; ¹H NMR (400 MHz CDCl₃), δ ppm 8.28 - 8.25 (d, *J* = 9.2 Hz, 1 H), 7.99 (br s, 1 H), 7.47 - 7.45 (d, *J* = 9.6 Hz, 1H), 1.54 (s, 9H).

### Step 2: synthesis of compound 6-2

To a solution of compound 6-1 (4.60 g, 20.0 mmol) in THF (300 mL) was added NaH (961 mg, 24.0 mmol, purity: 60.0%) at 0 °C. The mixture was stirred at 0 °C for 20 min, and then MeI (3.70 g, 26.0 mmol, 1.62 mL) was added at 0 °C. The resulting mixture was stirred at 25 °C for 40 min. After LC-MS showed that compound 6-1 had been completely consumed, the reaction mixture was quenched with 100 mL of water at 0 °C, and extracted with ethyl acetate (150 mL × 3). The combined organic layer was dried over [Na₂SO₄], filtered, and concentrated under reduced pressure to give a residue. The residue was purified by column chromatography (SiO₂, petroleum ether/ethyl acetate = 20/1) to give compound 6-2 (3.00 g, 12.3 mmol, yield: 61.4%) as a white solid. MS m/z (ESI): 244[M+H]⁺; ¹H NMR (400 MHz CDCl₃), δ ppm 8.13 - 8.11 (d, *J* = 9.2 Hz, 1H), 7.39 - 7.37 (d, *J* = 9.2 Hz, 1H), 3.55 (s, 3H), 1.55 (s, 9H).

### Step 3: synthesis of compound 6-3

To a solution of compound 6-2 (3.00 g, 12.3 mmol) and benzophenone imine (2.68 g, 14.77 mmol, 2.48 mL) in toluene (30.0 mL) were added BINAP (1.53 g, 2.46 mmol), Cs₂CO₃ (8.02 g, 24.6 mmol), and Pd₂(dba)₃ (1.13 g, 1.23 mmol) under nitrogen atmosphere, and the mixture was stirred at 110 °C for 16 h under nitrogen atmosphere. After LCMS showed that the starting material had been completely consumed, the reaction mixture was concentrated under reduced pressure to remove the solvent to give compound 6-3 (5.00 g, crude) as a yellow oil. MS m/z (ESI): 389[M+H]⁺.

### Step 4: synthesis of compound 6-4

Compound 6-3 (5.00 g, 4.38 mmol, purity: 34.0%) was dissolved in THF (100 mL), HCl (1 M, 10.0 mL) was added, and the mixture was stirred at 25 °C for 30 min. After LC-MS showed that compound 6-3 had been completely consumed, a sodium carbonate solution (5.00 mL) was added to the reaction mixture. The resulting mixture was extracted with ethyl acetate (10.0 mL × 3). The organic phase was dried over anhydrous sodium sulfate and filtered, and the filtrate was concentrated under reduced pressure. The residue was purified by column chromatography (SiO₂, petroleum ether/ethyl acetate = 10/1-4/1) to give compound 6-4 (800 mg, 3.57 mmol, yield: 81.5%) as a yellow solid. MS m/z (ESI): 244[M+H]⁺; ¹H NMR (400 MHz CDCl₃), δ ppm 7.33 - 7.31 (d, *J* = 9.2 Hz, 1H), 6.76 - 6.74 (d, *J* = 2.4 Hz, 1H), 6.26 (s, 2H), 3.19 (s, 2H), 1.39 (s, 7H).

### Step 5: synthesis of compound 6-5

Compound 6-4 (500 mg, 2.23 mmol) was dissolved in 4 M HCl/1,4-dioxane (2.23 mmol, 1 mL), and the mixture was stirred at 25 °C for 2 h. After LC-MS showed that compound 6-4 had been completely consumed, the reaction mixture was concentrated under reduced pressure to give compound 6-5 (276 mg, 2.22 mmol, yield: 99.7%) as a white solid. MS m/z (ESI): 125[M+H]⁺.

### Step 6: synthesis of compound 6-6

To a solution of fragment 1 (600 mg, 1.07 mmol) and compound 6-5 (264 mg, 2.13 mmol) in ACN (10.0 mL) were added T₃P (2.04 g, 3.20 mmol, 1.90 mL, purity: 50.0%) and Et₃N (1.08 g, 10.6 mmol, 1.48 mL), and the mixture was stirred at 40 °C for 2 h. After LC-MS showed that fragment 1 had been completely consumed, the reaction mixture was adjusted to pH 5-6 with a sodium carbonate solution at 0 °C, and then diluted with 20.0 mL of water and extracted with ethyl acetate (30.0 mL × 3). The organic layer was dried over anhydrous sodium sulfate and filtered, and the filtrate was concentrated under reduced pressure to give compound 6-6 as a yellow solid. MS m/z (ESI): 769 [M+H]⁺.

### Step 7: synthesis of compound 6

To a solution of compound 6-6 (600 mg, 897 µmol) in methanol (5.00 mL) was added sodium methoxide (484 mg, 8.97 mmol), and the mixture was stirred at 25 °C for 2 h. After LC-MS showed that compound 6-6 had been completely consumed, the reaction mixture was subjected to extraction and liquid separation in 10.0 mL of an aqueous ammonium chloride solution and 20.0 mL of an ethyl acetate solution. The organic phase was concentrated under reduced pressure to give a residue. The residue was purified by column chromatography (SiO₂, DCM:MeOH = 20/1-10/1) to give compound 6 (200 mg, 321 µmol, yield: 35.8%) as a white solid. MS m/z (ESI): 623[M+H]⁺; ¹H NMR (400 MHz CD₃OD), δ ppm 7.75 - 7.73 (d, *J* = 8.4 Hz, 2H), 7.46 - 7.41 (m, 3H), 7.36 - 7.38 (d, *J* = 9.2 Hz, 1H), 7.08 - 7.02(m, 3H), 5.47 - 5.45 (d, *J* = 10.4 Hz, 2H), 4.49 - 4.45 (br d, *J* = 14.8 Hz, 2H), 3.75 (s, 3H), 3.02 (s, 3H), 2.75 (s, 6H).

### Example 9. Synthesis of Compound 7

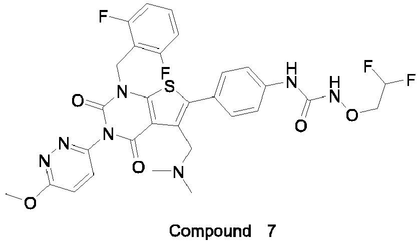

### Synthetic route:

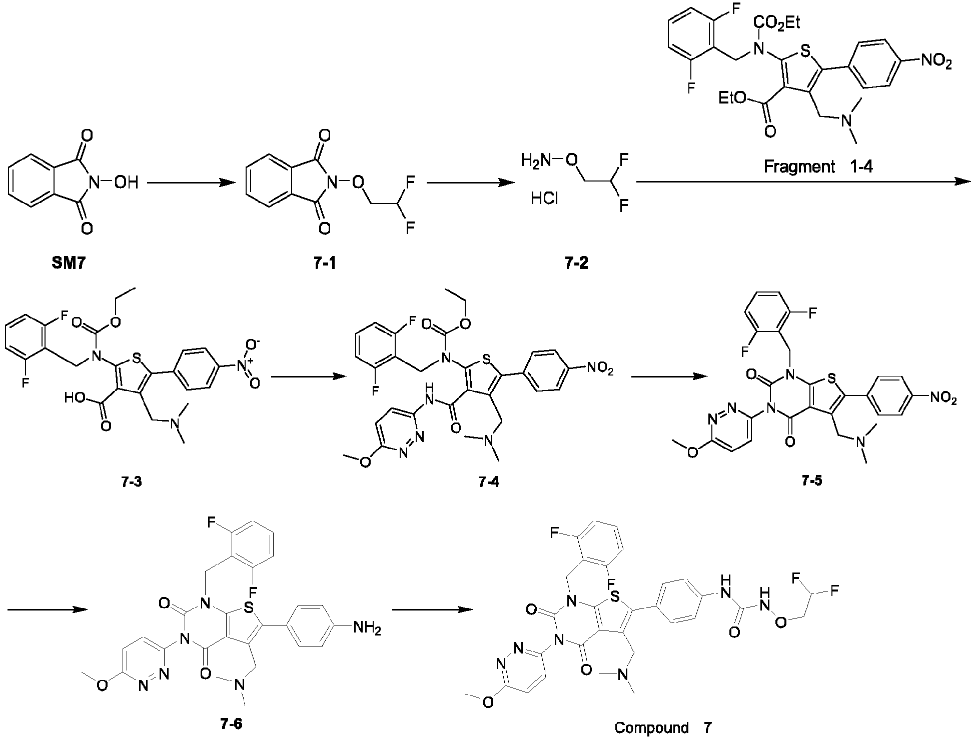

### Step 1: synthesis of compound 7-1

To SM7 (10.0 g, 61.3 mmol), 2,2-difluoroethanol (5.03 g, 61.3 mmol), and tetrahydrofuran (100 mL) was added PPh₃ (19.2 g, 73.5 mmol) at 0 °C, and the mixture was stirred at 25 °C for 20 h. After LC-MS showed that SM7 had been completely consumed, the reaction mixture was concentrated under reduced pressure to give a residue. The residue was purified by column chromatography (SiO₂, petroleum ether/ethyl acetate = 4/1) to give compound 7-1 (4.00 g, 17.6 mmol, 28.7%) as a white solid. MS m/z (ESI): 228[M+H]⁺; ¹H NMR (400 MHz CDCl₃), δppm 7.89 - 7.86 (m, 1H), 7.80 - 7.78 (m, 2H), 6.38 - 6.09 (m, 1H), 4.42 - 4.35 (m, 2H).

### Step 2: synthesis of compound 7-2

Compound 7-1 (4.00 g, 17.6 mmol) and NH₂NH₂·H₂O (2.01 g, 39.3 mmol, 1.95 mL, purity: 98.0%) was reacted in CH₂Cl₂ (100 mL). After LC-MS showed that compound 7-1 had been completely consumed, the reaction mixture was filtered, and the filtrate was washed with 10.0 mL of water. The combined organic layer was adjusted to pH 1 with 4 M HCl/1,4-dioxane (5.00 mL) and filtered to give compound 7-2 (2.00 g, 14.9 mmol, yield: 85.0%) as a white solid. MS m/z (ESI): 134[M+H]⁺; ¹H NMR (400 MHz DMSO-d6), δppm 11.3 (s, 2H), 6.51 - 6.22 (m, 1H), 4.36 - 4.28 (t, *J* = 29.6 Hz, 1H).

### Step 3: synthesis of compound 7-3

NaOH (3.29 g, 82.1 mmol) was added to H₂O (40.0 mL) to give 2 N NaOH. The solution was added to a solution of fragment 1-4 (9.00 g, 16.4 mmol) in EtOH (243 mL). The mixture was stirred at 60 °C for 6 h. After LC-MS showed that fragment 1-4 had been completely consumed, 1 N HCl (81.0 mL) was added at 0 °C, and the mixture was concentrated under vacuum. The residue was dissolved in diethyl ether (100 mL) and toluene (100 mL), and the whole was concentrated under vacuum. The residue was diluted with absolute ethanol (100 mL) and filtered, and the filtrate was concentrated under vacuum. To the residue was added methyl t-butyl ether (100 mL), and the mixture was concentrated under vacuum to give compound 7-3 (8.00 g, 13.8 mmol, yield: 84.3%) as a yellow solid. MS m/z (ESI): 520 [M+H]⁺.

### Step 4: synthesis of compound 7-4

To compound 7-3 (7.00 g, 13.4 mmol) and 3-amino-6-methoxypyridazine (3.37 g, 26.9 mmol) in ACN (100 mL) were added T₃P (25.7 g, 40.4 mmol, 24.0 mL, purity: 50.0%) and Et₃N (3.41 g, 33.6 mmol, 4.69 mL), and the mixture was stirred at 40 °C for 2 h. After LC-MS showed that compound 7-3 had been completely consumed, the reaction mixture was adjusted to pH 5-6 with a sodium carbonate solution at 0 °C, and then diluted with 100 mL of water and extracted with ethyl acetate (150 mL × 3). The organic layer was dried over anhydrous sodium sulfate and filtered, and the filtrate was concentrated under reduced pressure to give compound 7-4 (7.70 g, crude) as a yellow solid. MS m/z (ESI): 627 [M+H]⁺.

### Step 5: synthesis of compound 7-5

To a solution of compound 7-4 (7.20 g, 11.4 mmol) in methanol (200 mL) was added sodium methoxide (6.21 g, 114.9 mmol), and the reaction mixture was stirred at 25 °C for 2 h. After LC-MS showed that compound 7-4 had been completely consumed, the reaction mixture was partitioned between an aqueous ammonium chloride solution (10.0 mL) and ethyl acetate (200 mL). The organic phase was concentrated under reduced pressure to give compound 7-5 (4.30 g, crude) as a yellow solid. MS m/z (ESI): 581[M+H]⁺; ¹H NMR (400 MHz DMSO-d6), δppm 8.34 - 8.32 (d, *J* = 8.40 Hz, 2H), 8.00 - 07.97 (d, *J* = 8.80 Hz, 2H), 7.76 - 7.74 (d, *J* = 9.20 Hz, 2H),7.51 - 7.44 (m, 1H), 7.17 - 7.13 (t, *J* = 16.4 Hz, 2H), 5.43 - 5.21 (m, 2H), 4.09 (s, 3H), 3.73 - 3.66 (d, *J* = 27.2 Hz, 2H), 2.08 (s, 6H).

### Step 6: synthesis of compound 7-6

Compound 7-5 (4.70 g, 8.10 mmol) was dissolved in EtOH (500 mL), Pd/C (800 mg, 1.62 mmol) was added, and the mixture was stirred at 25 °C for 1 h. After LC-MS showed that compound 7-5 had been completely consumed, the reaction mixture was filtered and concentrated under reduced pressure to give a residue. The residue was purified by column chromatography (SiO₂, dichloromethane:MeOH = 10/1) to give compound 7-6 (2.90 g, 5.27 mmol, yield: 65.0%) as a white solid. MS m/z (ESI): 551[M+H]⁺; ¹H NMR (400 MHz CD₃OD), δ7.66 - 7.63 (m, 1H), 7.41 - 7.36 (m, 2H), 7.17 - 7.15 (m, 2H), 7.03 - 6.99 (m, 2H), 6.75 - 6.73 (m, 2H), 5.38 (s, 2H), 4.16 (s, 3H), 3.91 - 3.83 (m, 2H), 2.04 (s, 6H).

### Step 7: synthesis of compound 7

To a solution of compound 7-6 (200 mg, 363 µmol) in dichloromethane (5.00 mL) were added DIPEA (94.8 mg, 733 µmol, 127 µL) and CDI (117 mg, 726 µmol). The mixture was stirred at 25 °C for 14 h and then cooled to 0 °C. To the mixture were added DIPEA (478 mg, 3.71 mmol, 645 µL) and compound 7-2 (485 mg, 3.63 mmol). The resulting mixture was stirred at 25 °C for 2 h. After LC-MS showed that compound 7-6 had been completely consumed, a sodium carbonate solution (5.00 mL) was added to the reaction mixture. The resulting mixture was extracted with ethyl acetate (10.0 mL × 3). The organic phase was dried over anhydrous sodium sulfate and filtered, and the filtrate was concentrated under reduced pressure. The residue was purified by column chromatography (SiO₂, dichloromethane:MeOH = 10/1) to give a crude product, which was further purified by pre-TLC (SiO₂, dichloromethane:MeOH = 10/1) to give compound 7 (10.0 mg, 14.8 µmol, yield: 4.09%) as a yellow solid. MS m/z (ESI): 674[M+H]⁺; ¹H NMR (400 MHz CD₃OD), δppm 7.67 - 7.63 (m, 3H), 7.43 - 7.37 (m, 3H), 7.03 - 6.99 (t, *J* = 16.4 Hz, 2H), 6.29 - 6.02 (m, 1H), 3.46 - 3.35 (m, 1H), 5.39 (s, 2H), 4.16 (s, 3H), 4.14 - 4.07 (m, 2H), 2.00 (s, 6H).

### Example 10: Synthesis of Compound 2A

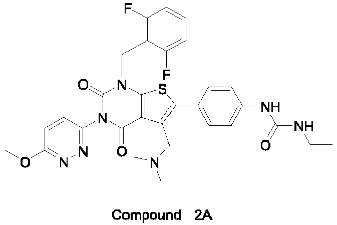

### Synthetic route:

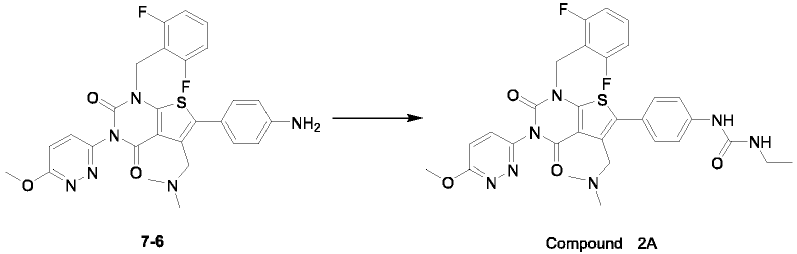

### Step 1: synthesis of compound 2A

To a solution of compound 7-6 (0.10 g, 182 µmol, 1.00 eq) in DMF (5 mL) was added Et₃N (110 mg, 1.09 mmol, 152 µL, 6.00 eq), and the mixture was stirred at 0 °C for 30 min. Then ethyl isocyanate (103 mg, 1.99 mmol, 403 µL, 28.0 eq) was added at 0 °C. The mixture was stirred at 50 °C for 17 h under N₂ atmosphere. Et₃N (55.1 mg, 545 µmol, 75.8 µL, 3.00 eq) and ethyl isocyanate (258 mg, 3.63 mmol, 288 µL, 20.00 eq) were additionally added, and the mixture was stirred at 50 °C for 5 h. Et₃N (55.1 mg, 545 µmol, 75.8 µL, 3.00 eq) and isocyanoethane (387 mg, 5.45 mmol, 431 µL, 30.0 eq) were additionally added, and the mixture was stirred at 50 °C for 5 h. After LCMS showed that compound 7-6 had been completely consumed, the mixture was poured into water (20 mL). The resulting mixture was extracted with ethyl acetate (15 mL × 3), washed with brine (10 mL × 2), dried over Na₂SO₄, and concentrated under reduced pressure to remove the solvent. The residue was purified by silica gel column chromatography (dichloromethane/methanol = 94/6) to give compound 2A (52.3 mg, 81.6 µmol, yield: 44.9%, purity: 97.0%) as a white solid. MS m/z (ESI): 622[M+H]⁺; ¹H NMR (400 MHz CD₃OD), δppm 8.32 (s, 1 H), 7.67 (d, *J* = 9.13 Hz, 1 H), 7.40 (d, *J* = 8.50 Hz, 2 H), 7.27 - 7.32 (m, 1 H), 7.24 (d, *J* = 9.13 Hz, 1 H), 6.95 (d, *J* = 8.38 Hz, 2 H), 6.83 - 6.91 (m, 2 H), 6.02 (br t, *J* = 5.32 Hz, 1 H), 5.08 - 5.36 (m, 2 H), 4.19 - 4.19 (m, 1 H), 4.19 (s, 2 H), 3.88 - 4.09 (m, 1 H), 3.30 (s, 2 H), 2.19 (s, 6 H), 1.16 (t, *J* = 7.19 Hz, 3 H). ¹⁹F NMR: (400 MHz, CDCl₃) δ -114.827 ppm.

### Example 11. Synthesis of Fragment 3

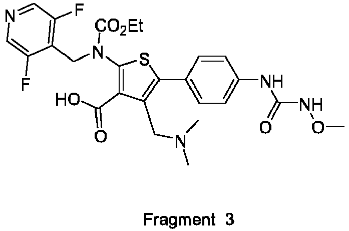

### Synthetic route:

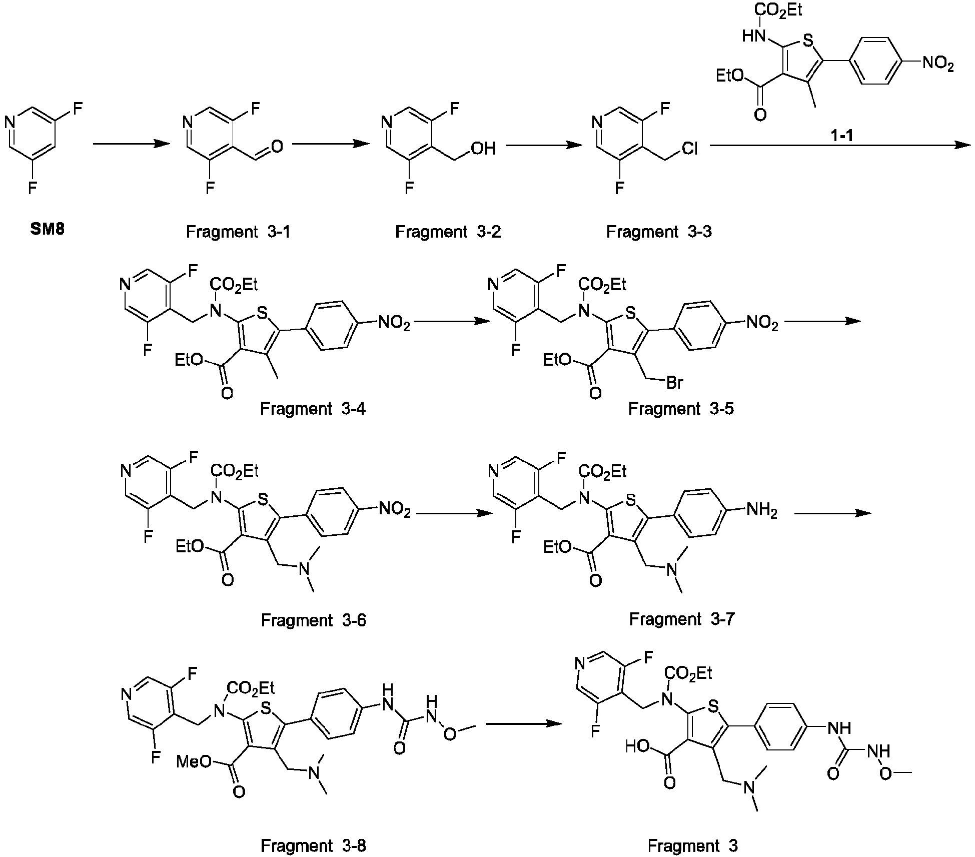

### Step 1: synthesis of fragment 3-1

SM8 (10.0 g, 86.9 mmol) was added to a solution (100 mL) of LDA (2 M, 43.5 mL) in THF (10.0 mL) at -70 °C. The mixture was stirred at -70 °C for 30 h, and then a methyl formate solution (10.4 g, 174 mmol, 10.5 mL, 2 eq) was added. The mixture was stirred at -70 °C for 1 h, and then stirred at 20 °C for 1 h. After TLC (petroleum ether:ethyl acetate = 3:1, Rf = 0.43) indicated that the reaction had been completed, the mixture was poured into aq. NaHCO₃ (100 mL) after cooling to 25 °C. The resulting mixture was extracted with ethyl acetate (50.0 mL × 3) and concentrated under reduced pressure to give fragment 3-1 (8.00 g, 55.9 mmol, 64.3%) as a yellow solid. ¹H NMR (400 MHz CDCl₃), δ ppm 8.55 (s, 2 H), 10.41 (s, 1 H).

### Step 2: synthesis of fragment 3-2

To a solution of fragment 3-1 (8.00 g, 55.9 mmol, 1 eq) in MeOH (100 mL) was added a solution of NaBH₄ (3.17 g, 83.9 mmol, 1.5 eq) in MeOH (10 mL) at 0 °C. The mixture was stirred at 20 °C for 2 h. After LCMS showed that the reaction had been completed, the reaction was quenched with water (50 mL) and concentrated under reduced pressure to remove the methanol. The residue was extracted with ethyl acetate (100 mL × 3). The combined organic phase was washed with brine (30.0 mL), dried over anhydrous Na₂SO₄, and concentrated under reduced pressure. The residue was slurried with petroleum ether:ethyl acetate = 10:1 (100 mL) at 25 °C for 1 h to give fragment 3-2 (6.21 g, 42.8 mmol, yield: 77.6%) as a yellow solid. MS m/z (ESI): 146[M+H]⁺; ¹H NMR (400 MHz CDCl₃), δ ppm 2.64 (br.s, 1 H), 4.84 (s, 2 H), 8.35 (s, 2 H).

### Step 3: synthesis of fragment 3-3

Compound fragment 3-2 (6.21 g, 42.8 mmol, 1 eq) was dissolved in CH₂Cl₂ (125 mL) under N₂ atmosphere. DIPEA (8.30 g, 64.2 mmol, 11.2 mL, 1.5 eq) and MsCl (6.00 g, 52.4 mmol, 4.05 mL, 1.22 eq) were added separately. The mixture was heated to 25 °C and stirred at 25 °C for 16 h. After LC-MS showed that the reaction had been completed, 50 mL of toluene was added twice. The resulting mixture was concentrated. The residue was purified by column chromatography (SiO₂, petroleum ether/ethyl acetate = 30/1-3/1) to give fragment 3-3 (2.46 g, 15.0 mmol, 35.1%) as a pale yellow liquid. MS m/z (ESI): 164[M+H]⁺; ¹H NMR (400 MHz CDCl₃), δ ppm 4.62 (s, 2 H), 8.38 (s, 2 H).

### Step 4: synthesis of fragment 3-4

To a solution of compound 1-1 (5.00 g, 13.2 mmol, 1 eq) in DMF (160 mL) were added KI (2.48 g, 14.9 mmol, 1.13 eq) and K₂CO₃ (2.06 g, 14.9 mmol, 1.13 eq). Then, to the mixture was added a solution of fragment 3-3 (2.45 g, 14.9 mmol, 1.13 eq) in DMF (12 mL). After LC-MS showed that the reaction had been completed, the resulting precipitate was filtered off, and the filtrate was concentrated under vacuum. The residue was diluted with ethyl acetate (70 mL) and water (70 mL), extracted with ethyl acetate (70 mL × 2), washed with water (70 mL) and brine (70 mL), dried over Na₂SO₄, and concentrated under vacuum. To the residue was added methyl t-butyl ether (35 mL), and the precipitate was collected by filtration and washed with methyl t-butyl ether (10.0 mL) to give fragment 3-4 (6.35 g, 12.5 mmol, yield: 95.1%) as a pale yellow solid. MS m/z (ESI): 506[M+H]⁺; ¹H NMR (400 MHz CDCl₃), δ ppm 1.19 - 1.37 (m, 6 H), 2.41 (s, 3 H), 4.13 - 4.33 (m, 4 H), 5.00 (s, 2 H), 7.54 (d, *J* = 8.80 Hz, 2 H), 8.28 (d, *J* = 8.80 Hz, 2 H), 8.34 (s, 2 H).

### Step 5: synthesis of fragment 3-5

Fragment 3-4 (6.35 g, 12.56 mmol, 1 eq), NBS (2.79 g, 15.70 mmol, 1.25 eq), and AIBN (206.28 mg, 1.26 mmol, 0.1 eq) were dissolved in ethyl acetate (75 mL), and the mixture was reacted at 80 °C for 2 h. To the mixture were added NBS (447 mg, 2.51 mmol, 0.2 eq) and AIBN (20.6 mg, 125.6 µmol, 0.01 eq). The resulting mixture was heated at 80 °C for 6 h. After LC-MS showed that the reaction had been completed, the reaction mixture was diluted with ethyl acetate (50 mL) and water (50 mL), extracted with ethyl acetate (30 mL × 2), washed with water (40 mL) and brine (40 mL), dried (Na₂SO₄), and concentrated under vacuum. The residue was purified by column chromatography (SiO₂, petroleum ether/ethyl acetate = 30/1-8/1) to give fragment 3-5 (4.63 g, 7.92 mmol, 63.07%) as a pale yellow solid. MS m/z (ESI): 584,586[M+H]⁺.

### Step 6: synthesis of fragment 3-6

Fragment 3-5 (4.63 g, 7.92 mmol, 1 eq), dimethylamine (1.94 g, 23.8 mmol, 2.18 mL, 3 eq, HCl), and DMF (20.1 mL) were added to a 100 mL single-necked round-bottomed flask at 25 °C. Then triethylamine (3.21 g, 31.7 mmol, 4.41 mL, 4 eq) was added, and the mixture was stirred for 2 h. After LC-MS showed that the reaction had been completed, the reaction was quenched by adding water (60 mL), extracted with ethyl acetate (60 mL × 3), and subjected to liquid separation. The organic phase was collected and concentrated by rotary evaporation under reduced pressure. The residue was purified by column chromatography (SiO₂, petroleum ether/ethyl acetate = 10/1-1/4) to give fragment 3-6 (3.05 g, 5.56 mmol, yield: 70.2%) as a pale yellow solid. MS m/z (ESI): 549[M+H]⁺; ¹H NMR (400 MHz CDCl₃), δ ppm 1.10 - 1.25 (m, 3 H), 1.31 (t, *J* = 7.20 Hz, 3 H), 2.08 (s, 6 H), 3.55 (s, 2 H), 4.14 - 4.30 (m, 4 H), 5.05 (s, 2 H), 7.66 (d, *J* = 7.66 Hz, 2 H), 8.27 (d, *J* = 8.80 Hz, 2 H), 8.33 (s, 2 H).

### Step 7: synthesis of fragment 3-7

To a solution of fragment 3-6 (3.05 g, 5.56 mmol, 1 eq) and HCl/1,4-dioxane (4 M, 2.78 mL, 2 eq) in EtOH (84.0 mL) was added Pd/C (0.99 g, 5.56 mmol, purity: 10%, 1.00 eq), and the mixture was hydrogenated under hydrogen atmosphere (15psi) at 25 °C for 1 h. After LC-MS showed that the reaction had been completed, the reaction mixture was filtered through celite, and the filtrate was diluted with a saturated aqueous NaHCO₃ solution (25 mL). The filtrate was concentrated under reduced pressure, and ethyl acetate (50 mL) and water (50 mL) were added. The resulting mixture was extracted with ethyl acetate (50 mL × 3). The combined organic phase was washed with saturated brine (50 mL), dried over Na₂SO₄, and concentrated under reduced pressure to give fragment 3-7 (2.74 g, yield: 95.0%) as a pale yellow oil, which was directly used in the next step without purification. MS m/z (ESI): 519[M+H]⁺; ¹H NMR (400 MHz CDCl₃), δ ppm 1.11 - 1.24 (m, 3 H), 1.31 (t, *J* = 7.20 Hz, 1 H), 1.28 - 1.34 (m, 3 H), 2.02 - 2.06 (m, 6 H), 3.43 - 3.60 (m, 2 H), 3.75 - 3.87 (m, 2 H), 4.15 - 4.31 (m, 4 H), 4.98 - 5.08 (m, 2 H), 6.66 - 6.72 (m, 2 H), 7.13 - 7.20 (m, 2 H), 8.27 - 8.33 (m, 2 H).

### Step 8: synthesis of fragment 3-8

To a solution of fragment 3-7 (2.74 g, 5.28 mmol, 1 eq) in CH₂Cl₂ (70 mL) were added *N*,*N-*diisopropylethylamine (2.80 g, 21.6 mmol, 3.77 mL, 4.1 eq) and CDI (3.43 g, 21.1 mmol, 4 eq). The mixture was stirred at 25 °C for 13 h. The mixture was cooled to 0 °C. To the mixture were added methoxyamine hydrochloride (4.42 g, 52.8 mmol, 10 eq, HCl) and *N*,*N-*diisopropylethylamine (6.82 g, 52.8 mmol, 9.20 mL, 10 eq). The resulting mixture was stirred at 25 °C for 8 h. After LC-MS showed that the reaction had been completed, the reaction mixture was washed with a saturated aqueous NaHCO₃ solution (80 mL). The organic layer was separated, and the aqueous layer was extracted with CH₂Cl₂ (50 mL × 2). The combined organic phase was washed with brine (100 mL), dried over Na₂SO₄, and concentrated under vacuum. The residue was purified by column chromatography (SiO₂, dichloromethane:methanol = 30/1-8/1) to give fragment 3-8 (2.82 g, 4.77 mmol, yield: 71.57%) as a pale yellow oil. MS m/z (ESI): 578[M+H]⁺; ¹H NMR (400 MHz DMSO-d6), δ ppm 1.00 - 1.22 (m, 6 H), 1.93 (s, 6 H), 3.42 (s, 2 H), 3.63 (s, 3 H), 4.07 (q, *J* = 7.20 Hz, 4 H), 4.97 (s, 2 H), 7.32 (d, *J* = 7.20 Hz, 2 H), 7.69 (d, *J* = 8.40 Hz, 2 H), 8.51 (s, 2 H), 9.06 (s, 1 H), 9.62 (s, 1 H).

### Step 9: synthesis of fragment 3

Sodium hydroxide (953 mg, 23.8 mmol, 5 eq) was added to water (12 mL) to form a 2 M sodium hydroxide solution. A 2 M NaOH solution was added to a solution of fraction 3-8 (2.82 g, 4.77 mmol, 1 eq) in EtOH (72 mL). The mixture was stirred at 60 °C for 2 h. After LC-MS showed that the reaction had been completed, 1 mol/L HCl (24 mL) was added at 0 °C. The resulting mixture was concentrated under vacuum. The residue was dissolved in ethanol (50 mL × 3) and toluene (50 mL × 3), and the mixture was concentrated under vacuum. The residue was diluted with absolute ethanol and filtered, and the filtrate was concentrated under vacuum. The residue was purified by high-performance liquid chromatography (neutral conditions, column: xate C18 150 × 40 mm × 10 µm, mobile phase: [water (NH₄HCO₃)-ACN], B%: 10%-50%, 10 min) to give fragment 3 (1.33 g, 2.36 mmol, yield: 49.5%) as a pale yellow solid. MS m/z (ESI): 564[M+H]⁺; ¹H NMR (400 MHz DMSO-d6), δ ppm 1.08 (t, *J* = 6.82 Hz, 3 H), 2.45 (s, 6 H), 3.63 (s, 3 H), 3.95 (s, 2 H), 4.03 (q, *J* = 6.82 Hz, 2 H), 4.95 (s, 2 H), 7.22 (d, *J* = 8.40 Hz, 2 H), 7.72 (d, *J* = 8.80 Hz, 2 H), 8.49 (s, 2 H), 9.2 (s, 1 H), 9.64 (s, 1 H).

### Example 12. Synthesis of Compound 8

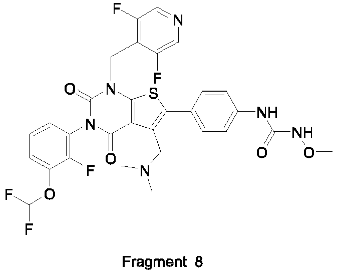

### Synthetic route:

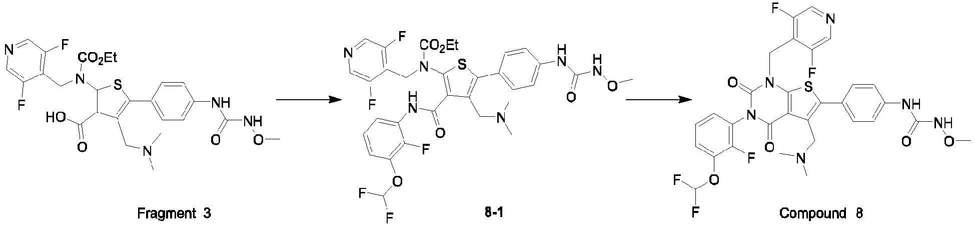

### Step 1: synthesis of compound 8-1

Fragment 3 (500 mg, 887.20 µmol, 1 eq) and 3-difluoromethoxy-2-fluoroaniline (282.86 mg, 1.60 mmol, 1.8 eq) were dissolved in ACN (10 mL), T₃P (1.69 g, 2.66 mmol, 1.58 mL, purity: 50%, 3 eq) and Et₃N (224.44 mg, 2.22 mmol, 308.72 µL, 2.5 eq) were added, and the mixture was stirred at 40 °C for 12 h. T₃P (1.69 g, 2.66 mmol, 1.58 mL, purity: 50%, 3 eq) and Et₃N (224.44 mg, 2.22 mmol, 308.72 µL, 2.5 eq) were added, and the mixture was stirred at 40 °C for 6 h. The reaction mixture was adjusted to pH 5-6 with a saturated sodium carbonate solution at 0 °C, and then diluted with 100.0 mL of water and extracted with ethyl acetate (100 mL × 3). The organic phase was dried over anhydrous sodium sulfate and filtered, and the filtrate was concentrated under reduced pressure. The filtrate was concentrated under reduced pressure to give compound 8-1 (600 mg, crude) as a yellow oil. MS m/z (ESI): 723[M+H]⁺.

### Step 2: synthesis of compound 8

Compound 8-1 (600 mg, 830.24 µmol, 1 eq) was dissolved in MeOH (10 mL), and NaOMe (448.53 mg, 8.30 mmol, 10 eq) was added. The mixture was stirred at 25 °C for 2 h. The reaction mixture was adjusted to pH 5-6 with a saturated sodium carbonate solution at 0 °C, and then diluted with 100.0 mL of water and extracted with ethyl acetate (100 mL × 3). The organic phase was dried over anhydrous sodium sulfate and filtered, and the filtrate was concentrated under reduced pressure. The residue was purified by column chromatography (SiO₂, DCM:methanol = 100/1-10/1) and further purified by pre-TLC (SiO₂, DCM:MeOH = 10:1) to give compound 8 (114 mg, 141.88 µmol, yield: 17.09%, purity: 96%) as a yellow solid. MS m/z (ESI): 677[M+H]⁺; ¹H NMR (400 MHz CD₃OD), δ ppm 8.43 (s, 2 H) 7.67 (d, *J* = 8.00 Hz, 2 H) 7.40 - 7.48 (m, 3 H) 7.25 - 7.39 (m, 2 H) 7.06 - 7.11 (m, 1 H) 6.90 (s, 1 H) 6.72 (s, 1 H) 5.32 - 5.61 (m, 2 H) 4.60 (br s, 1 H) 3.92 - 4.05 (m, 1 H) 3.55 - 3.80 (m, 4 H) 2.06 - 2.22 (m, 6 H).

### Example 13: Synthesis of Compound 8A

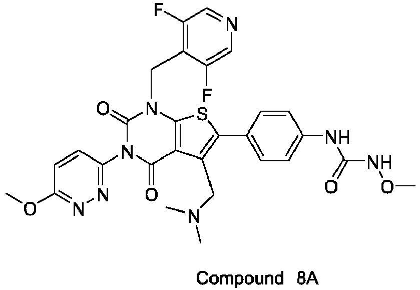

### Synthetic route:

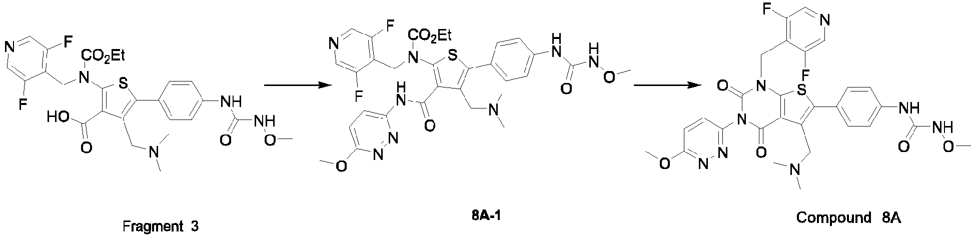

### Step 1: synthesis of compound 8A-1

To a solution of **fragment 3** (500 mg, 887.20 µmol, 1 eq) was added a solution of 3-amino-6-methoxypyridazine (555.07 mg, 4.44 mmol, 5 eq), T₃P (4.52 g, 7.10 mmol, 4.22 mL, purity: 50%, 8 eq), and DIEA (1.72 g, 13.31 mmol, 2.32 mL, 15 eq) in DMF (10 mL). The mixture was stirred at 25 °C for 12 h. The reaction mixture was adjusted to pH 5-6 with a saturated sodium carbonate solution at 0 °C, and then diluted with 100.0 mL of water and extracted with ethyl acetate (30 mL × 3). The organic phase was dried over anhydrous sodium sulfate, filtered, and concentrated under reduced pressure to give **compound 8A-1** (470 mg, crude) as a yellow oil. MS m/z (ESI): 671[M+H]⁺.

### Step 2: synthesis of compound 8A

To a solution of **compound 8A-1** (470 mg, 700.78 µmol, 1 eq) in MeOH (10 mL) was added NaOMe (378.58 mg, 7.01 mmol, 10 eq). The mixture was stirred at 25 °C for 16 h. After LCMS showed that the starting material had been completely consumed, and 47% of the desired MS had been detected, the mixture was poured into a saturated aq. NH₄Cl solution (50 mL). The resulting mixture was extracted with CH₂Cl₂ (50 mL × 2). The combined organic layer was dried over anhydrous Na₂SO₄ and concentrated under reduced pressure. The mixture was purified by high-performance liquid chromatography (pre-HPLC) (column: YMC Triart 30 × 150 mm × 7 µm; mobile phase: [water (ammonium hydroxide solution v/v)-ACN]; B%: 0%-60%, 40 min) to give **compound 8A** (70 mg, 104.56 µmol, yield: 14.92%, purity: 93.3%) as a white solid. MS m/z (ESI): 625[M+H]⁺; ¹H NMR (400 MHz CD₃OD), δ ppm 8.41 (s, 2 H) 7.62 - 7.70 (m, 3 H) 7.42 - 7.49 (m, 2 H) 7.37 (d, *J* = 8.0 Hz, 1 H) 5.45 (br s, 2 H) 4.59 (br s, 2 H) 4.16 (s, 3 H) 3.74 (s, 3 H) 2.08 (s, 6 H).

### Test Example 1. In Vitro Antagonistic Activity Screening

Cell: HEK293 cells stably expressing human GnRHR (WuXi AppTec (Shanghai) Co., Ltd.) Equipment: 384-well plate, Greiner; Vi-cell XR Cell Viability Analyzer, Beckman Coulter; incubator, Thermo.

Method: HEK293 cells stably expressing human GnRHR were seeded into a 384-well plate at a density of 20,000/well. Fluo-4 Direct^{™} No-wash Loading Buffer (Invitrogen) was added to each well the next day, and then the plate was put back into an incubator, incubated at 37 °C for 50 min, and then incubated at room temperature for 10 min. Test compounds were formulated in DMSO. 10 µL of each of the compounds was added to each well, with test concentrations being 1000 nM, 250 nM, 62.5 nM, 15.6 nM, 3.9 nM, 976.6 pM, 244.1 pM, 61.0 pM, 15.3 pM, and 3.8 pM. Fluorescence values were determined using a FLIPR Tetra high throughput real-time fluorescence detection and assay system. Then 10 µL of Leuprolide acetate was added, and fluorescence values were determined. IC₅₀ and IC₉₀ of the compounds were calculated using Prism. The results are shown in Table 1.

**Table 1. In vitro antagonistic activity of the compounds of the present invention against human GnRHR**

| Compound | Functional IC₅₀ (nM) | Functional IC₉₀ (nM) |
|---|---|---|
| Relugolix¹ | 1.91 | 7.00 |
| 1 | 2.18 | 9.97 |
| 1-9 | 0.67 | 4.05 |
| 2 | 1.01 | 7.55 |
| 2A | 1.84 | 8.19 |
| 3 | 1.70 | 7.76 |
| 3A | 4.71 | 43.08 |
| 4 | 12.04 | 92.27 |
| 5 | 1.61 | 8.41 |
| 6 | 2.03 | 65.44 |
| 7 | 2.34 | 15.32 |
| 8 | 10.94 | 97.35 |
| 8A | 671.6 | - |
| 1. A compound prepared and disclosed in Example 83 of CN100360538C. | | |

Conclusion: multiple compounds of the present invention have a significant inhibitory effect on human GnRHR.

### Test Example 2. In Vitro Binding Screening

Cell: Chem-1 cells stably expressing human GnRHR (Eurofins Panlabs Discovery Services) Reagent: [¹²⁵I] [D-Trp6]-LH-RH, PerkinElmer; [D-Trp6]-LH-RH, Abcam.

Equipment: MicroBeta2 LumiJET system, PerkinElmer.

Method: Chem-1 cell membranes stably expressing human GnRHR were resuspended in HEPES at pH 7.4. 6 µg of human GnRHR cell membranes were added to each well and incubated with 0.05 nM [¹²⁵I]-[D-Trp6]-LH-RH and each of the test compounds at 25 °C for 60 min. The compounds were each dissolved in DMSO, with test concentrations being 500 nM, 62.5 nM, 7.81 nM, 0.97 nM, 0.12 nM, 15 pM, and 1.9 pM. 1 µM [D-Trp6]-LH-RH was used in the non-specific binding assay. Cell membranes were collected by filtration under vacuum and washed before liquid scintillation. The binding IC₅₀ and IC₉₀ of the compounds were calculated using Prism. The results are shown in Table 2.

**Table 2. In vitro binding activity of the compounds of the present invention against human GnRHR**

| Name of the compounds | Binding IC₅₀ (nM) | Binding IC₉₀ (nM) |
|---|---|---|
| Relugolix | 0.25 | 1.42 |
| 1 | 0.57 | 5.33 |
| 2 | 0.64 | 2.67 |
| 2A | 0.87 | 13.20 |
| 3 | 0.26 | 0.97 |
| 5 | 0.38 | 2.97 |
| 6 | 0.55 | 4.96 |
| 7 | 0.22 | 1.40 |

Conclusion: the compounds of the present invention have significant binding ability to human GnRHR.

### Test Example 3. hERG Test

Cell: CHO cells stably expressing a human hERG potassium channel (WuXi AppTec (Shanghai) Co., Ltd.)

Reagent: physiological solution (pH = 7.4): 140 mM NaCl, 4 mM KCl, 2 mM CaCl₂, 1 mM MgCl₂, 5 mM Glucose, 10 mM HEPES, Osmolarity: about 298 mOsm; external solution (pH = 7.4): 80 mM NaCl, 4 mM KCl, 2 mM CaCl₂, 1 mM MgCl₂, 5 mM Glucose, 60 mM NMDG, 10 mM HEPES, Osmolarity: about 289 mOsm; internal solution (pH = 7.4): 10 mM NaCl, 10 mM KCl, 110 mM KF, 10 mM HEPES, EGTA: 10 nM, Osmolarity: about 280 mOsm.

Equipment: QPatch high-throughput automated patch clamp system, Sophion.

Method: CHO cells stably expressing a human hERG potassium channel were digested with TrypLE and resuspended in a physiological solution for later use. The single-cell high-impedance sealing and whole-cell mode formation processes were automatically completed by Qpatch. The clamping voltage for the cells was -80 mV. Then a voltage of -50 mV was applied for 80 ms to subtract (current) leakage, and a voltage of +20 mV was applied for 4800 ms to open the hERG channel. The voltage was then reduced to -50 mV and maintained for 5000 ms. This process was used for data collection and analysis. Finally, the voltage was returned to the clamping voltage (-80 mV, 1000 ms). This process was repeated every 20,000 ms. 40 µL of solvent control (external solution + DMSO) was added first, and the test was performed for 300 ms to collect baseline data. Then, 40 µL of each of the test compounds was added at various concentrations, and the test was performed for no less than 300 ms. The compounds were each dissolved in DMSO, with test concentrations being 0.30 µM, 1.00 µM, 3.00 µM, 10.00 µM, and 30.00 µM. The IC₅₀ data for the hERG test were obtained by analyzing using DataControl, Excel2013, and Prism. The results are shown in Table 3.

**Table 3. Inhibition of human hERG potassium channel by the compounds of the invention**

| Name of the compounds | hERG IC₅₀ (µM) |
|---|---|
| Relugolix | 4.14 |
| 1 | 4.31 |
| 1-9 | 1.37 |
| 2 | 25.79 |
| 2A | 6.07 |
| 4 | 4.44 |
| 5 | 7.81 |
| 6 | 20.68 |
| 7 | 4.93 |
| 8A | >30 |

Conclusion: the inhibitory activity of compounds 1, 2, 2A, 4, 5, 6, 7, and 8A on the human hERG potassium channel is equal to or less than that of Relugolix, indicating that the compounds of the present invention have lower cardiac toxicity.

### Test Example 4. Evaluation of Metabolic Stability in Liver Microsomes

### Liver microsome: rat liver microsome and human liver microsome

Reagent: NADPH, Roche Diagnostics Gmbh; ACN, Merck Chemical; Tolbutamide, Sigma. Instrument: water purification equipment, Millipore; electronic balance, METTLER TOLEDO; highspeed benchtop centrifuge, Thermo; water bath thermostatic oscillator, Shanghai Yiheng Technical Co., Ltd.; vortex oscillator, Thermo; LC-MS/MS, AB SCIEX.

Method: the metabolic stability of test substances was evaluated by using SD rat and human liver microsomes. The test substances were incubated with liver microsomes from different species by using NADPH under a water bath at 37 °C. A cold acetonitrile solution containing a Tolbutamide internal standard substance was added at a specified time point to terminate the reaction. The samples were pre-treated and then subjected to semi-quantitative analysis by LC-MS/MS. The retention times of the analytes and Tolbutamide internal standard, the acquisition of chromatograms, and the integration of chromatograms were processed using software Analyst (AB Sciex, Framingham, Massachusetts, USA). The *in vitro* elimination rate constant Ke of the samples was obtained by converting the ratio of the peak area of the samples to the peak area of the internal standard into the residual percentage, and the *in vitro* clearance rate and the half-life of the test samples were calculated. The results are shown in Table 4.

**Table 4. Results for metabolic stability of the compounds of the present invention in rat and human liver microsomes**

| Species | Compound | Residual content at 60 min | T_{1/2} | CL_{int(mic)} | CLᵢₙₜ₍ₗᵢᵥₑᵣ₎ |
|---|---|---|---|---|---|
| | | % | min | µL/min/mg protein | mL/min/kg |
| Rat | Relugolix | 58.0 | 84.5 | 16.4 | 29.5 |
| | 2 | 61.0 | 87.7 | 15.8 | 28.4 |
| | 3 | 34.5 | 39.8 | 34.8 | 62.6 |
| | 4 | 89.9 | >186 | <7.50 | <13.5 |
| | 5 | 98.7 | >186 | <7.50 | <13.5 |
| | 6 | 57.1 | 71.4 | 19.4 | 34.9 |
| | 7 | 47.0 | 55.0 | 25.2 | 45.4 |
| Human | Relugolix | 86.4 | 239 | 5.80 | 5.22 |
| | 2 | 83.8 | 257 | 5.40 | 4.86 |
| | 3 | 55.5 | 71.4 | 19.4 | 17.5 |
| | 4 | 104 | >186 | <7.50 | <6.75 |
| | 5 | 89.0 | >186 | <7.50 | <6.75 |
| | 6 | 95.9 | >186 | <7.50 | <6.75 |
| | 7 | 75.5 | 169 | 8.20 | 7.38 |

Conclusion: compounds 4 and 5 showed low clearance in the SD rat liver microsome, the remaining compounds all showed moderate clearance; compound 3 showed moderate clearance in the human liver microsome, and the remaining compounds all showed low clearance.

### Test Example 5. Evaluation of Permeability of Test Substances and Transporter Substrate

### Cell: Caco-2 cells

Instrument: water purification equipment, ELGA LabWate; biosafety cabinet, NUAIRE; thermostatic CO₂ incubator, Thermo; microplate reader, PerkinElmer; LC-MS/MS, AB SCIEX.

Method: A Caco-2 monolayer cell model was used to determine the two-way permeability of the test substances and evaluate whether the test substances were transported by the efflux of a transporter. In the experiment, Caco-2 cells were seeded into a 96-well cell culture plate and cultured for 28 consecutive days for transport assay. The test substances were each administered on two sides. After being incubated for 120 min, the samples at the apical end A, at the basal end B, and with cell lysis were collected and quantitatively determined for the content of the test substances by using a liquid chromatography-tandem mass spectrometry (LC/MS/MS) method. The apparent permeability coefficient (Papp) in the direction from the apical end to the basal end (A-B) and in the direction from the basal end to the apical end (B-A) and the efflux ratio were calculated from the concentrations of the test substances. The results are shown in Table 5.

**Table 5. Results for permeability of the compounds of the present invention in Caco-2 cells**

| Compound | Mean _{Papp} (10⁻⁶ cm/s) | | Efflux ratio |
|---|---|---|---|
| | A to B | B to A | |
| Relugolix | 0.00716 | 13.8 | 1932 |
| 1 | 0.984 | 32.5 | 33 |
| 1-9 | 0.0317 | 42.2 | 1332 |
| 2 | 0.0142 | 4.19 | 294 |
| 2A | 0.00816 | 9.65 | 1182 |
| 3 | 0.126 | 18.0 | 143 |
| 4 | 0.0933 | 29.4 | 316 |
| 5 | 0.0110 | 4.58 | 417 |
| 6 | 0.0504 | 0.504 | 10.0 |
| 7 | 0.0101 | 15.6 | 1549 |

Conclusion: most of the compounds of the present invention show improved permeability in the Caco-2 cell experiment as compared to Relugolix, with compound 2 showing a nearly 6-fold improvement in the efflux ratio.

### Test Example 6. Study on Pharmacokinetics and Tissue Distribution in SD Rats

Rat: SD rats, male, 200-250 g

Instrument: water purification equipment, Millipore; electronic balance, METTLER TOLEDO; highspeed benchtop centrifuge, Thermo; water bath thermostatic oscillator, Shanghai Yiheng Technical Co., Ltd.; vortex oscillator, Thermo; LC-MS/MS, AB SCIEX.

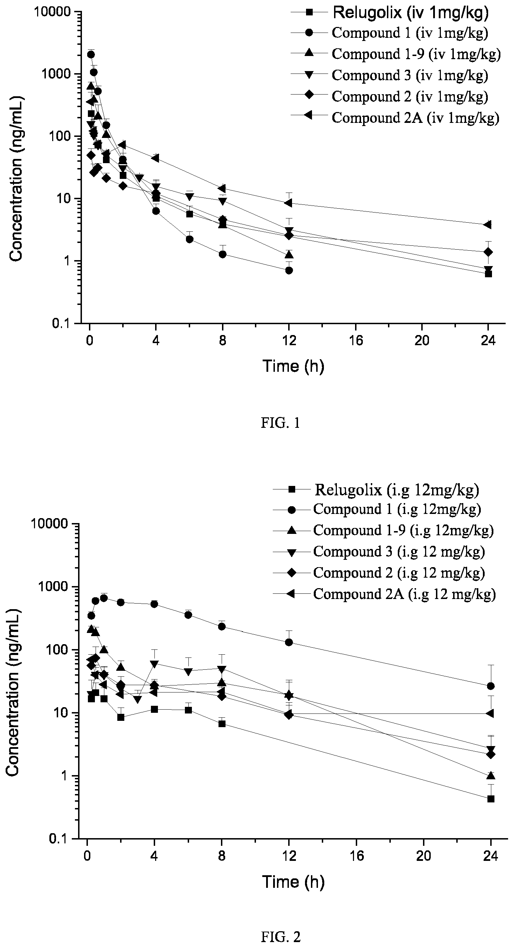

Method: all compounds were formulated in DMSO and 20% solutol. In the pharmacokinetic experiment, the administration dose for the intravenous group (i.v) was 1 mg/kg, and the administration dose for the intragastric group (i.g) was 12 mg/kg, each group containing 3 rats. Blood was collected from the orbit of the rats before (0 h) the administration and at set time points after administration. In the tissue distribution experiment, the rats were subjected to intragastric administration at a dose of 12 mg/kg. After the administration, the rats were anesthetized and dissected at set time points, heart blood was collected, and the pituitary was taken by heart perfusion, with 3 rats at each time point. The plasma was placed in a heparinized EP tube and centrifuged at 13,500 rpm for 10 min to isolate the plasma. The pituitary was homogenized with water in a certain proportion. The plasma and pituitary homogenates were pre-treated and then analyzed by LC-MS/MS to determine the concentration of the test substances in the plasma and the pituitary. The plasma drug concentration data were processed in a non-compartmental model using WinNonlin^{™} Version 6.3 (Pharsight, Mountain View, CA) pharmacokinetic software. The following pharmacokinetic parameters were calculated using a linear-log trapezoidal method: elimination phase half-life (T_{1/2}), mean residence time of drug *in vivo* from time point 0 to the last time point (MRT₀₋ₗₐₛₜ), area under the time-plasma concentration curve from time point 0 to the last time point (AUC₀₋ₗₐₛₜ), initial concentration (C₀) or maximum concentration Cₘₐₓ, and absolute bioavailability F. The results are shown in FIGs. 1-2 and Tables 6-1, 6-2, and 6-3.

**Table 6-1. Pharmacokinetic parameters in SD rats after intravenous administration**

| Dosage | Compound | T_{1/2} (h) | C₀ (ng/ml) | AUCₗₐₛₜ (h*ng/ml) | MRTₗₐₛₜ (h) |
|---|---|---|---|---|---|
| i.v 1 mg/kg | Relugolix | 3.64±1.25 | 339±36 | 212±61 | 2.01±0.92 |
| | 1 | 2.79±0.87 | 2828±454 | 992±226 | 0.539±0.041 |
| | 1-9 | 2.60±0.90 | 822±274 | 460±163 | 1.16±0.13 |
| | 2 | 6.39±1.08 | 68.8±21.7 | 150±12 | 5.41±0.47 |
| | 2A | 6.64±3.10 | 521±322 | 487±150 | 4.14±1.83 |
| | 3 | 4.34±1.00 | 198±4 | 271±49 | 3.83±0.63 |

**Table 6-2. Pharmacokinetic parameters in SD rats after intragastric administration**

| Dosage | Compound | T_{1/2} (h) | Tₘₐₓ (h) | Cₘₐₓ (ng/ml) | AUCₗₐₛₜ (h*ng/ml) | MRTₗₐₛₜ (h) | F (%) |
|---|---|---|---|---|---|---|---|
| i.g 12 mg/kg | Relugolix | 3.88±0.57 | 0.330±0.140 | 21.6±8.3 | 146±37 | 5.68±0.17 | 5.74 |
| | 1 | 4.86±3.05 | 1.13±0.63 | 664±123 | 5319±1207 | 6.02±0.93 | 44.7 |
| | 1-9 | 4.33±2.91 | 0.31±0.13 | 220±32 | 604±165 | 4.84±2.10 | 10.9 |
| | 2 | 4.31±1.81 | 0.38±0.14 | 78.5±36.4 | 357±85 | 6.13±1.10 | 19.9 |
| | 2A | 18.0±15.9 | 0.25±0.00 | 69.9±15.1 | 370±109 | 9.20±2.30 | 6.33 |
| | 3 | 4.11 | 6.00±2.83 | 82.7±10.1 | 596±231 | 7.30±1.13 | 18.3 |

**Table 6-3. Pituitary tissue distribution in SD rats after intragastric administration 12 mg/kg)**

| Compound | Time | Plasma | Pituitary | Pituitary/plasma |
|---|---|---|---|---|
| Relugolix | 1h | 22.2±10.2 | 944±591 | 42.4 |
| | 4h | 16.3±5.52 | 1246±777 | 76.3 |
| | 12h | 3.17±2.53 | 510±213 | 161 |
| 2 | 1h | 39.5±14.0 | 2250±474 | 56.9 |
| | 4h | 22.8±2.5 | 1841±636 | 80.8 |
| | 12h | 9.70±3.10 | 1254±494 | 129 |
| 2A | 1h | 32.2±18.0 | 280±80 | 8.7 |
| | 4h | 13.3±5.1 | 279±70 | 21 |
| | 12h | 5.59±1.55 | 333±90 | 59.6 |

Conclusion:
(1) In the intragastric administration group, the Cₘₐₓ of compound 1 was 30.7-fold and 3.02-fold higher than that of Relugolix and compound 1-9, respectively; the exposure AUCₗₐₛₜ of compound 1 was 36.4-fold and 8.80-fold higher than that of Relugolix and compound 1-9, respectively. The absolute bioavailability of compound 1 was 7.79-fold and 4.10-fold higher than that of Relugolix and compound 1-9, respectively. The *in vivo* pharmacokinetic properties of compound 1 were significantly better than those of Relugolix and compound 1-9.
(2) In the intragastric group, the Cₘₐₓ, AUCₗₐₛₜ, and absolute bioavailability of compound 2 were 3.63-fold, 2.45-fold, and 3.47-fold higher than those of Relugolix, respectively, and were 1.12-fold, 0.96-fold, and 3.14-fold higher than those of compound 2A, respectively. The concentrations of compound 2 in the pituitary at 1 h, 4 h, and 12 h were 2.38-fold, 1.48-fold, and 2.46-fold higher than those of Relugolix, respectively, and were 8.04-fold, 6.60-fold, and 3.77-fold higher than those of compound 2A, respectively.
(3) In the intragastric group, the Cₘₐₓ, AUCₗₐₛₜ, and absolute bioavailability of compound 3 were 3.83-fold, 4.08-fold, and 3.19-fold higher than those of Relugolix, respectively.

## Claims

1. A compound of formula (I), a pharmaceutically acceptable salt thereof or a stereoisomer thereof, 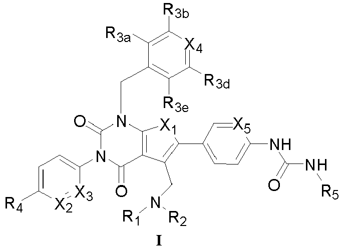 wherein:
X₁ is independently selected from O or S;
X₂ and X₃ are each independently selected from CR₄ or N;
X₄ is independently selected from CR_{3c} or N;
X₅ is independently selected from CR₆ or N;
R₁ is independently selected from H, D, C₁-C₆ alkyl, C₃-C₆ cycloalkyl, and C₁-C₆ haloalkyl;
R₂ is independently selected from H, D, C₁-C₆ alkyl, and C₁-C₆ alkyl substituted with R₂ₐ-O-;
R₂ₐ is independently selected from C₁-C₆ haloalkyl;
R₃ₐ, R_{3b}, R_{3c}, R_{3d}, and R₃ₑ are each independently selected from H, D, halogen, C₁-C₆ alkyl, C₁-C₆ haloalkyl, and C₃-C₆ cycloalkyl;
R₄ is independently selected from H, D, halogen, -CN, -NO₂, -NH₂, -OH, -SH, -COOH, C₁-C₆ alkyl,
C₁-C₆ haloalkyl, C₁-C₆ alkoxy, C₁-C₆ haloalkoxy, C₁-C₆ alkylthio, C₁-C₆ alkylamino, and C₃-C₆ cycloalkyl;
R₅ is independently selected from H, D, C₁-C₆ alkyl, C₃-C₆ cycloalkyl, C₁-C₆ haloalkyl, C₁-C₆ alkoxy,
C₃-C₆ cycloalkoxy, and C₁-C₆ haloalkoxy;
R₆ is independently selected from H, D, C₁-C₆ alkyl, C₃-C₆ cycloalkyl, C₁-C₆ haloalkyl, C₁-C₆ alkoxy,
and C₁-C₆ haloalkoxy.

2. A compound of formula (III), a pharmaceutically acceptable salt thereof or a stereoisomer thereof, 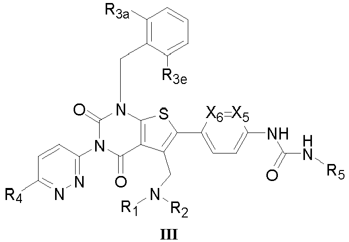 wherein:
X₅ and X₆ are independently selected from CR₆ or N, and X₅ and X₆ are not both CR₆ or N;
R₁, R₂, R₃ₐ, R₃ₑ, R₄, and R₅ are as defined in claim 1.

3. The compound, the pharmaceutically acceptable salt thereof or the stereoisomer thereof according to claim 1 or 2, wherein R₁ is selected from H, D, methyl, ethyl, n-propyl, isopropyl, n-butyl, isobutyl, s-butyl, t-butyl, n-pentyl, isopentyl, neopentyl, 1-ethylpropyl, n-hexyl, isohexyl, 1,1-dimethylbutyl, 2,2-dimethylbutyl, 3,3-dimethylbutyl, 2-ethylbutyl, cyclopropyl, cyclobutyl, cyclopentyl, cyclohexyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, and pentachloroethyl.

4. The compound, the pharmaceutically acceptable salt thereof or the stereoisomer thereof according to any one of claims 1-3, wherein R₂ is selected from C₁-C₆ alkyl or C₁-C₆ alkyl substituted with R₂ₐ-O-; R₂ is independently selected from methyl, ethyl, and propyl, and R₂ₐ is independently selected from fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, and pentachloroethyl.

5. The compound, the pharmaceutically acceptable salt thereof or the stereoisomer thereof according to any one of claims 1-4, wherein R₃ₐ, R_{3b}, R_{3c}, R_{3d}, and R₃ₑ are each independently selected from H, D, F, Cl, Br, I, methyl, ethyl, *n*-propyl, isopropyl, *n*-butyl, isobutyl, *s*-butyl, *t*-butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, cyclopropyl, cyclobutyl, cyclopentyl, and cyclohexyl.

6. The compound, the pharmaceutically acceptable salt thereof or the stereoisomer thereof according to any one of claims 1-5, wherein R₄ is independently selected from H, D, F, Cl, Br, I, -CN, -NO₂, -NH₂, -OH, -SH, -COOH, methyl, ethyl, *n*-propyl, isopropyl, *n*-butyl, isobutyl, *s*-butyl, *t*-butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, 2-ethylbutyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, *n-*propoxy, isopropoxy, *n*-butoxy, *s*-butoxy, *t*-butoxy, *n*-pentyloxy, *S*-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, pentachloroethoxy, methylthio, ethylthio, *n*-propylthio, isopropylthio, *n*-butylthio, *s*-butylthio, *t*-butylthio, *n*-pentylthio, *S-*pentylthio, hexylthio, 2-ethylbutylthio, methylamino, dimethylamino, ethylamino, diethylamino, *n*-propylamino, isopropylamino, *n*-butylamino, *s*-butylamino, *t*-butylamino, *n*-pentylamino, *S-*pentylamino, hexylamino, 2-ethylbutylamino, cyclopropyl, cyclobutyl, cyclopentyl, and cyclohexyl.

7. The compound, the pharmaceutically acceptable salt thereof or the stereoisomer thereof according to any one of claims 1-6, wherein R₅ is independently selected from H, D, methyl, ethyl, *n*-propyl, isopropyl, *n*-butyl, isobutyl, *s*-butyl, *t*-butyl, *n*-pentyl, isopentyl, neopentyl, 1-ethylpropyl, *n-*hexyl, isohexyl, 1,1-dimethylbutyl, 2,2-dimethylbutyl, 3,3-dimethylbutyl, 2-ethylbutyl, cyclopropyl, cyclobutyl, cyclopentyl, cyclohexyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, *n-*propoxy, isopropoxy, *n*-butoxy, *s*-butoxy, *t*-butoxy, *n*-pentyloxy, *S*-pentyloxy, hexyloxy, 2-ethylbutoxy, cyclopropyloxy, cyclobutoxy, cyclopentyloxy, cyclohexyloxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, and pentachloroethoxy; R₆ is independently selected from methyl, ethyl, *n-*propyl, isopropyl, *n*-butyl, isobutyl, *s*-butyl, *t*-butyl, *n*-pentyl, isopentyl, neopentyl, 1-ethylpropyl, *n*-hexyl, isohexyl, 1,1-dimethylbutyl, 2,2-dimethylbutyl, 3,3-dimethylbutyl, 2-ethylbutyl, cyclopropyl, cyclobutyl, cyclopentyl, cyclohexyl, fluoromethyl, chloromethyl, difluoromethyl, dichloromethyl, trifluoromethyl, trichloromethyl, 2,2-difluoroethyl, 2,2-dichloroethyl, 3,3,3-trifluoroethyl, 3,3,3-trichloroethyl, pentafluoroethyl, pentachloroethyl, methoxy, ethoxy, *n-*propoxy, isopropoxy, *n*-butoxy, *s*-butoxy, *t*-butoxy, n-pentyloxy, *S*-pentyloxy, hexyloxy, 2-ethylbutoxy, fluoromethoxy, chloromethoxy, difluoromethoxy, dichloromethoxy, trifluoromethoxy, trichloromethoxy, 2,2-difluoroethoxy, 2,2-dichloroethoxy, 3,3,3-trifluoroethoxy, 3,3,3-trichloroethoxy, pentafluoroethoxy, and pentachloroethoxy.

8. A compound as shown below, a pharmaceutically acceptable salt thereof or a stereoisomer thereof, selected from:

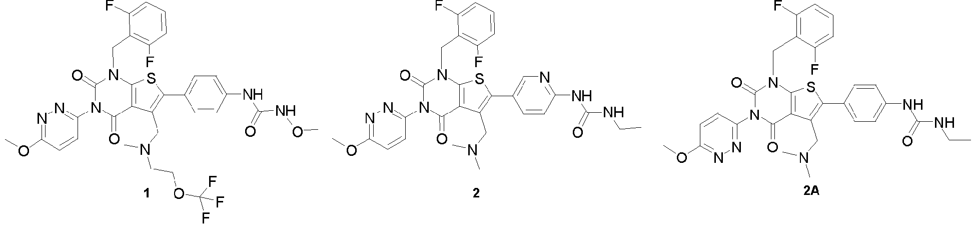

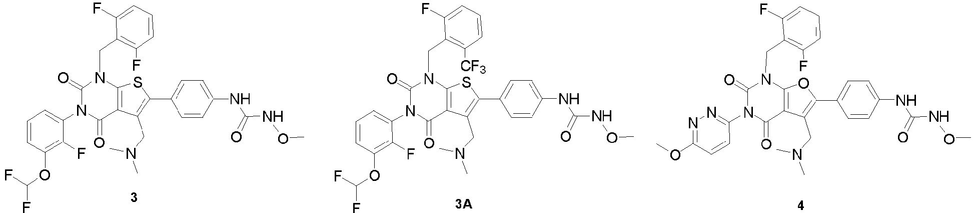

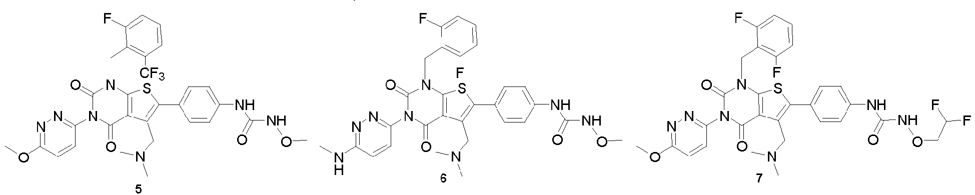

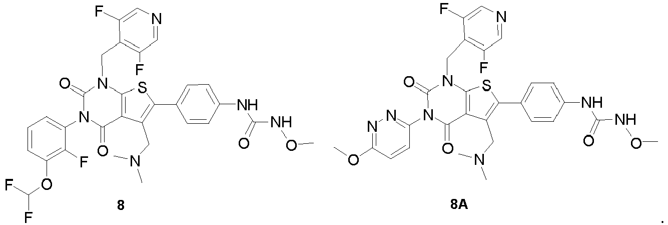

9. A pharmaceutical composition comprising the compound, the pharmaceutically acceptable salt thereof or the stereoisomer thereof according to any one of claims 1-8, and a pharmaceutically acceptable carrier.

10. Use of the compound, the pharmaceutically acceptable salt thereof or the stereoisomer thereof according to any one of claims 1-8, or the pharmaceutical composition according to claim 9 in preparing a medicament for the treatment of a sex hormone-dependent disease.

11. The use according to claim 10, wherein the sex hormone-dependent disease includes: sex hormone-dependent cancer, bone metastases of sex hormone-dependent cancer, prostatic hypertrophy, prostate cancer, uterine cancer, breast cancer, pituitary cancer, hysteromyoma, endometriosis, metrofibroma, precocious puberty, amenorrhea, premenstrual syndrome, dysmenorrhoea, multilocular ovarian syndrome, polycystic ovary syndrome, acne, alopecia, Alzheimer's disease, infertility, irritable bowel syndrome, benign or malignant tumors that are hormone-independent and sensitive to LH-RH (luteinizing hormone-releasing hormone), or flushing.

12. The use of the compound, the pharmaceutically acceptable salt thereof or the stereoisomer thereof according to any one of claims 1-8, or the pharmaceutical composition according to claim 9 in preparing a reproduction modulator, a contraceptive, or an ovulation inducer; or use thereof as a medicament for the prevention of postoperative recurrence of sex hormone-dependent cancer.
